(19) Europäisches Patentamt
European Patent Office
Office européen des brevets

(11) Publication number: **0 308 559 A2**

(12) **EUROPEAN PATENT APPLICATION**

(21) Application number: **87309154.0**

(22) Date of filing: **15.10.87**

(51) Int. Cl.4: **C07D 501/57 , A61K 31/545**

The title of the invention has been amended (Guidelines for Examination in the EPO, A-III, 7.3).

(30) Priority: **24.09.87 GB 8722477**

(43) Date of publication of application: **29.03.89 Bulletin 89/13**

(84) Designated Contracting States: **BE CH DE FR GB IT LI NL**

(71) Applicant: **BEECHAM GROUP PLC**
**Beecham House Great West Road**
**Brentford Middlesex TW8 9BD(GB)**

(72) Inventor: **Branch, Clive Leslie**
**Beecham Pharmaceuticals Brockham Park**
**Betchworth Surrey RH3 7AJ(GB)**
Inventor: **Kaura, Arun Chandar**
**Beecham Pharmaceuticals Brockham Park**
**Betchworth Surrey RH3 7AJ(GB)**

(74) Representative: **Lockwood, Barbara Ann et al**
**Beecham Pharmaceuticals Biosciences**
**Research Centre Great Burgh Yew Tree**
**Bottom Road**
**Epsom Surrey KT18 5XQ(GB)**

(54) **Cephalosporins, process for their preparation, pharmaceutical compositions containing them and intermediates.**

(57) β-Lactam antibiotics are disclosed which have the formula (IA) or are pharmaceutically acceptable salts or pharmaceutically acceptable in vivo hydrolysable esters thereof:

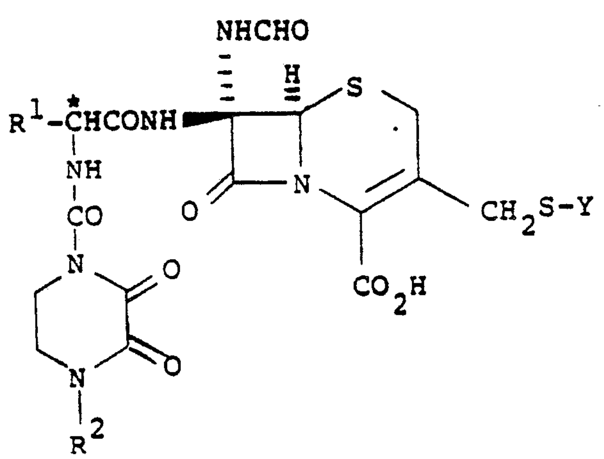

(IA)

wherein Y is a 1-(optionally substituted amino)-1H-tetrazol-5-yl, 4-(optionally substituted amino)-1,2,4-triazol-3-yl, or 2-(optionally substituted amino)-1,3,4-thiadiazol-5-yl group; $R^1$ is phenyl, substituted phenyl, cyclohexenyl, cyclohexadienyl, or an optionally substituted 5- or 6- membered heterocyclic ring containing up to three hetero atoms selected from oxygen, nitrogen and sulphur; and $R^2$ is $C_{1-8}$ alkyl; and also the

EP 0 308 559 A2

use thereof.

Processes for the preparation of the compounds are disclosed together with intermediates for use therein.

## NOVEL COMPOUNDS

This invention relates to a class of novel β-lactam derivatives, which have antibacterial activity and are of value in the treatment of infections in animals especially mammals including man caused by a wide range of organisms, particularly Gram-negative organisms. The invention also relates to a process for the preparation of such compounds, intermediates for use in the preparation of the compounds and to pharmaceutical compositions comprising the antibacterially active compounds.

European Patent Application Number 82303821.1 (Publication No.0 071 395) discloses a class of β-lactam antibiotics having an α-formamido (formamidyl) substituent on the carbon atom adjacent to the carbonyl group of the β-lactam ring.

It has now been found that, within this class of compounds, there exist cephalosporin derivatives with outstanding antibacterial properties.

Accordingly the present invention provides a compound of formula (I) or a salt thereof:

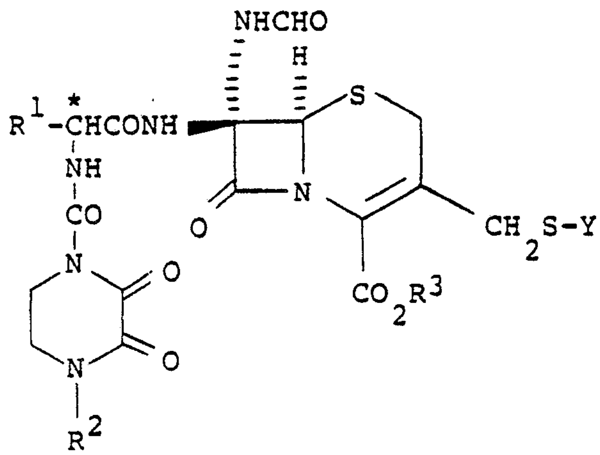

(I)

wherein Y is a group of formula (a), (b) or (c):

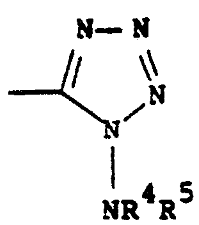

(a)

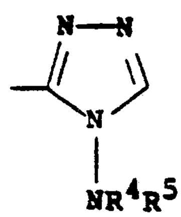

(b)

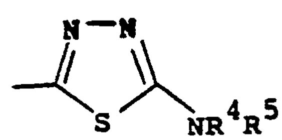

(c)

wherein $R^4$ is hydrogen, optionally substituted $C_{1-8}$ alkyl, formyl, optionally substituted $C_{1-6}$ alkylcarbonyl, optionally substituted $C_{1-6}$ alkoxycarbonyl, arylcarbonyl, aryl $C_{1-6}$ alkylcarbonyl, aryl $C_{1-6}$ alkoxycarbonyl or a group of the formula:

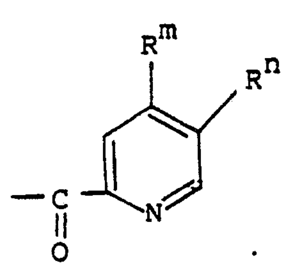

wherein $R^m$ and $R^n$ are hydroxy or protected hydroxy; $R^5$ is hydrogen or $C_{1-8}$ alkyl; or $R^4$ and $R^5$ together form a group $-(CH_2)_n-$ wherein n is an integer having the value 2 to 7, or a group $-(CH_2)_p X (CH_2)_q-$ wherein X is O, S or a group NR wherein R is H or $C_{1-6}$ alkyl and p and q are integers which may be the same or different, each having the value 1 to 5 and wherein p + q has the value 2 to 6; or $R^4$ and $R^5$ together form a $C_{1-6}$ alkylidene or arylalkylidene group containing up to 6 carbon atoms in the alkylidene moiety; $R^1$ is phenyl, substituted phenyl, cyclohexenyl, cyclohexadienyl, or a 5- or 6-membered heterocyclic ring containing up to three hetero-atoms selected from oxygen, sulphur or nitrogen, optionally substituted with hydroxy, amino, halogen, $C_{1-6}$ alkylamino, di($C_{1-6}$ alkyl)amino, or $C_{1-6}$ alkoxy; $R^2$ is $C_{1-8}$ alkyl; and $R^3$ is hydrogen or a readily removable carboxy protecting group.

In compounds of formula (I), the formamido group can exist in conformations wherein the hydrogen atoms of the -NH-CHO moiety are cis- or trans-; of these the cis conformation normally predominates.

When used herein the term 'aryl' includes phenyl and naphthyl each optionally substituted with up to five, preferably up to three, groups selected from halogen, $C_{1-6}$ alkyl, phenyl, $C_{1-6}$ alkoxy, hydroxy($C_{1-6}$)-alkyl, mercapto($C_{1-6}$)alkyl, halo($C_{1-6}$)alkyl, mercapto, hydroxy, amino, nitro, carboxy, $C_{1-6}$ alkoxycarbonyl, $C_{1-6}$ alkoxycarbonyl($C_{1-6}$)alkyl, $C_{1-6}$ alkylcarbonyloxy, formyl, or $C_{1-6}$ alkylcarbonyl groups.

The term "halogen' refers to fluorine, chlorine, bromine and iodine.

The carbon atom marked * in formulae herein is asymmetric and thus compounds of formula (I) may exist as two optically active diastereoisomers. In general the isomer prepared from the D-side chain exhibits the highest antibacterial activity and accordingly the D compound or the DL mixtures are preferred, with the D compound being particularly preferred.

The compounds of formula (I) with the preferred D-side-chain can be separated from a mixture of both diastereoisomers by conventional methods, or prepared from intermediates that bear a D- side chain.

Suitably the substituted.phenyl group for $R^1$ is a phenyl group substituted with up to three groups selected from $C_{1-6}$ alkyl, phenyl, halogen, amino, nitro, hydroxy, $C_{1-6}$ alkylamido, carbamoyl, carboxy, $C_{1-6}$ alkoxycarbonyl, aryloxycarbonyl, halo ($C_{1-6}$) alkyl, hydroxy($C_{1-6}$)alkyl, oxo($C_{1-6}$)alkyl, $C_{1-6}$ alkylcarbonyl, arylcarbonyl, $C_{1-6}$ alkylamino, di($C_{1-6}$) alkylamino, or sulphonamido, any amino and hydroxy groups being optionally protected.

The optional protecting groups for, hydroxy or amino groups attached to the phenyl ring in $R^1$ are suitably readily cleaved and include in vivo hydrolysable groups as well as groups which may be cleaved by conventional chemical or enzymatic methods.

A comprehensive discussion of the ways in which hydroxy and amino groups may be protected and methods for cleaving the resulting protected derivatives are given, for example, in "Protective Groups in Organic Synthesis" by T. W. Greene (Wiley-Interscience, New York, 1981). Particularly suitable protecting groups include those which, when protecting a hydroxy group in $R^1$ afford esters or carbonates (both of which may be in vivo hydrolysable), ethers (including silyl ethers) and acetals (for example tetrahydropyranyloxy derivatives, sometimes described as 'THP ethers'). When $R^1$ is aminophenyl, suitable protecting groups include, for example, those that afford amides and carbamates.

When $R^1$ is dihydroxyphenyl, for example 3,4-dihydroxyphenyl, it will be understood that one or both of the hydroxy groups may be protected. When both hydroxy groups are protected it will be understood that a different protecting group may be used for each hydroxy group, although, more conveniently, the protecting groups used will be the same.

Examples of suitable protecting groups for a hydroxy group in $R^1$ include formyl and optionally substituted $C_{1-6}$ alkylcarbonyl and arylcarbonyl groups such as acetyl, chloroacetyl, dichloroacetyl and benzoyl; optionally substituted $C_{1-6}$ alkoxycarbonyl and aryl $C_{1-6}$ alkoxycarbonyl, for example ethoxycarbonyl, trimethylsilylethoxycarbonyl, benzyloxycarbonyl and 4-nitrobenzyloxycarbonyl; and optionally substituted $C_{2-6}$ alkenyloxycarbonyl such as allyloxycarbonyl.

Further examples of suitable protecting groups for a hydroxy group in $R^1$ include aryl, aryl $C_{1-6}$ alkyl, optionally substituted $C_{1-6}$ alkyl, optionally substituted $C_{2-6}$ alkenyl, optionally substituted $C_{3-7}$ cycloalkyl,and silyl groups.

Some examples of optional substituents in groups mentioned hereinabove as being optionally substituted include up to three groups (which may be the same or different) chosen from halogen, hydroxy, $C_{1-6}$ alkoxy, $C_{1-6}$ alkylthio, cyano, nitro, carboxy, carboxylic acid $C_{1-6}$ alkyl ester, carbamoyl, amino, mono ($C_{1-6}$) alkylamino, and di ($C_{1-6}$) alkylamino.

Preferred alkyl protecting groups for a hydroxy group in $R^1$ include, for example, methyl or ethyl, optionally substituted with ($C_{1-6}$) alkoxy or ($C_{1-6}$) alkylthio, for example with methoxy, ethoxy, or methylthio. Further useful protecting groups are methoxyethoxymethyl and (trimethylsilyl)ethoxymethyl. In addition, when $R^1$ is 3,4-dihydroxyphenyl, the hydroxy groups may be protected by an alkylene bridge so that $R^1$ becomes, for example, 3,4-[1,1-dimethyl(methylenedioxy)]phenyl.

Preferred aryl $C_{1-6}$ alkyl protecting groups for a hydroxy group in $R^1$ include benzyl and 4-nitrobenzyl.

Preferred silyl protecting groups may be substituted with $C_{1-6}$ alkyl and/or phenyl groups and include, for example, trimethylsilyl, t-butyldimethylsilyl, t-butyldiphenylsilyl, triethylsilyl, isopropyldimethylsilyl, triphenylmethyldimethylsilyl and the like. The resulting silyl ethers may be cleaved by methods known in the art, such as those described by T. W. Greene (loc. cit.) or by M. Lalonde and T.H. Chan in Synthesis, 1985 (September), pages 817-845 and references therein.

It will be understood that hydroxy groups mentioned herein as 'protected' may suitably be protected by groups mentioned hereinabove as suitable for protecting hydroxy groups present in $R^1$.

Particularly preferred hydroxy protecting groups are acetyl and trimethylsilyl.

In one favoured group of compounds according to the invention $R^1$ is phenyl, 3-hydroxyphenyl, 3-tri($C_{1-6}$ alkyl)silyloxyphenyl, 3-($C_{1-6}$ alkylcarbonyloxy) phenyl, 3-($C_{1-6}$ alkyloxycarbonyloxy)phenyl, 3-(benzyloxycarbonyloxy)phenyl, 3-(4-nitrobenzyloxycarbonyloxy)phenyl, 3-aminophenyl, 4-hydroxyphenyl, 4-tri($C_{1-6}$ alkyl)silyloxyphenyl, 4-($C_{1-6}$ alkylcarbonyloxy)phenyl, 4-($C_{1-6}$ alkyloxycarbonyloxy) phenyl, 4-(benzyloxycarbonyloxy)phenyl, 4-(4-nitrobenzyloxycarbonyloxy)phenyl, 4-aminophenyl, 3,4-dihydroxyphenyl, 3,4-bis[tri ($C_{1-6}$ alkyl)silyloxy] phenyl, 3,4-di($C_{1-6}$ alkylcarbonyloxy)phenyl, 3,4-di ($C_{1-6}$ alkyloxycarbonyloxy)phenyl, 3,4-di(benzyloxycarbonyloxy)phenyl, or 3,4-di (4-nitrobenzyloxycarbonyloxy)phenyl.

Preferred groups for $R^1$ include phenyl, 3,4-dihydroxyphenyl, 3,4-diacetoxyphenyl, 4-hydroxyphenyl, and 4-acetoxyphenyl.

Suitable values for the group $R^2$ include $C_{1-6}$ alkyl, for example methyl, ethyl, propyl and butyl.

A preferred value for the group $R^2$ is ethyl.

Suitable values for the group $R^4$ include hydrogen; $C_{1-6}$ alkyl, for example methyl and ethyl; formyl; arylcarbonyl, for example phenylcarbonyl optionally substituted in the phenyl ring with up to two hydroxy groups or protected hydroxy groups, $C_{1-4}$ alkylcarbonyl optionally substituted by halogen, for example acetyl and chloroacetyl; benzyl optionally substituted in the phenyl ring with up to two hydroxy groups or protected hydroxy groups; benzylcarbonyl; and benzyloxycarbonyl.

Preferred values for the group $R^4$ include hydrogen, methyl, chloroacetyl, formyl, 3,4-diacetoxybenzoyl and 3,4-dihydroxybenzoyl.

Particularly preferred values for the group $R^4$ hydrogen, 3,4-dihydroxybenzoyl and formyl.

Advantageously the group $R^4$ may also be a group of the formula:

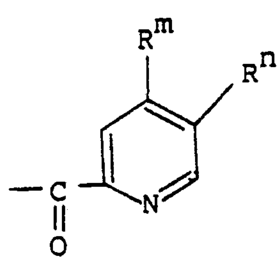

as hereinabove defined. It will be understood that $R^m$ and $R^n$ may be the same or different. Preferably $R^m$ and $R^n$ are both hydroxy.

When $R^m$ and $R^n$ are hydroxy, will be appreciated that compounds bearing the (4,5-dihydroxypyridin-2-yl)carbonyl group may exist in different tautomeric forms, for example that having a (5-hydroxy-4-oxo-1,4-dihydropyridin-2-yl)carbonyl group of formula:

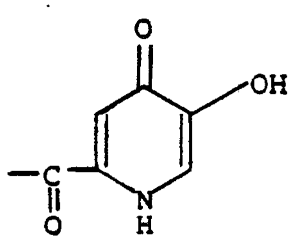

All such tautomers are included within the scope of this invention.

Suitable values for the group $R^5$ include hydrogen and $C_{1-6}$ alkyl.

Preferably the group $R^5$ is hydrogen.

When the groups $R^4$ and $R^5$ together form a $C_{1-6}$ alkylidene group the alkylidene group suitably contains 2 to 6 carbon atoms, a preferred $R^4$ $R^5$ group being isopropylidene.

Non-pharmaceutically acceptable salts of the compound of formula (I) wherein $R^3$ is hydrogen are primarily of use as intermediates in the preparation of a compound of formula (I) wherein $R^3$ is hydrogen or a pharmaceutically acceptable salt thereof. Salts within compounds of formula (I) may be prepared by salt exchange in conventional manner.

Similarly, carboxy-protected derivatives of formula (I), i.e. those compounds of formula (I) wherein $R^3$ is a readily removable carboxy protecting group, may be used as intermediates in the preparation of a compound of the formula (I) wherein $R^3$ is hydrogen, or a pharmaceutically acceptable salt thereof. Included within the scope of readily removable carboxy protecting groups for $R^3$ are ester groups including pharmaceutically acceptable in vivo hydrolysable ester groups.

Since the β-lactam antibiotic compounds of the present invention are intended for use in pharmaceutical compositions, it will be readily appreciated that preferred compounds within formula (I) are pharmaceutically acceptable, i.e. are compounds of formula (IA) or pharmaceutically acceptable salts or pharmaceutically acceptable in vivo hydrolysable esters thereof:

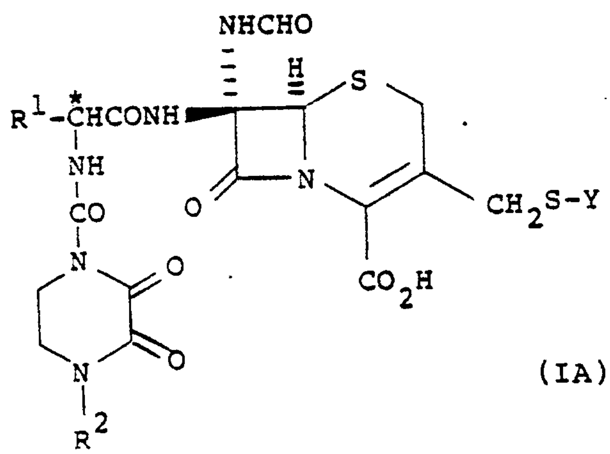

(IA)

wherein $R^1$, $R^2$, Y, and * are as defined with respect to formula (I).

Since the β-lactam antibiotic compounds of the present invention are intended for use in pharmaceutical compositions, it will be further understood that they are each provided in substantially pure form, for example at least 50% pure, more suitably at least 75% pure and preferably at least 95% pure (% are on a wt/wt basis). Impure preparations of the compounds may be used for preparing the more pure forms used in the pharmaceutical compositions. Although the purity of intermediate compounds of the present invention is less critical it will readily be understood that the substantially pure form is preferred as for the β-lactam antibiotic compounds. Preferably, whenever possible, the compounds of the present invention are obtained in crystalline form.

When some of the compounds of this invention are allowed to crystallise, or are recrystallised, from organic solvents, solvent of crystallisation may be present in the crystalline product. This invention includes within its scope such solvates. Similarly, some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation.

Suitable readily removable carboxyl protecting groups ior the group $-CO_2R^3$ include groups forming ester derivatives of the carboxylic acid, including in vivo hydrolysable esters. The derivative is preferably one which may readily be cleaved.

Suitable ester-forming carboxyl-protecting groups are those which may be removed under conventional conditions. Such groups for $R^3$ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl, methoxymethyl, a silyl, stannyl or phosphorus-containing group, an oxime radical of formula $-N=CHR^6$ where $R^6$ is aryl or heterocyclic, or an in vivo hydrolysable ester radical such as defined below.

A carboxyl group may be regenerated from any of the above esters by usual methods appropriate to the particular $R^3$ group, for example, acid- and base-catalysed hydrolysis, or by enzymically-catalysed hydrolysis, or by hydrogenolysis under conditions wherein the remainder of the molecule is substantially unaffected.

Examples of suitable pharmaceutically acceptable in vivo hydrolysable ester groups include those which break down readily in the human body to leave the parent acid or its salt. Suitable ester groups of this type include those of part formula (i), (ii), (iii) and (iv):

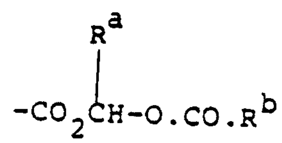

(i)

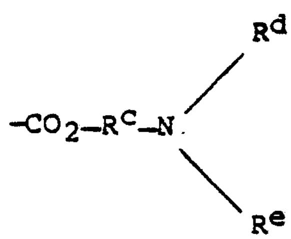

(ii)

$-CO_2CH_2-OR^f$ (iii)

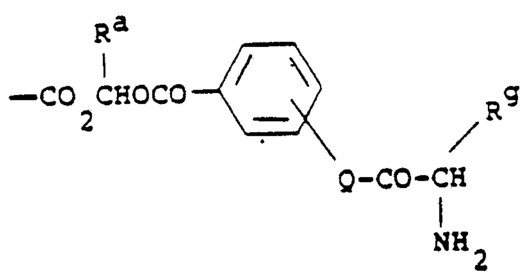

(iv)

wherein $R^a$ is hydrogen, $C_{1-6}$ alkyl, $C_{3-7}$ cycloalkyl, methyl, or phenyl, $R^b$ is $C_{1-6}$ alkyl, $C_{1-6}$ alkoxy, phenyl, benzyl, $C_{3-7}$ cycloalkyl, $C_{1-6}$ alkyl $C_{3-7}$ cycloalkyl, 1-amino $C_{1-6}$ alkyl, or 1-($C_{1-6}$ alkyl)amino $C_{1-6}$ alkyl; or $R^a$ and $R^b$ together form a 1,2-phenylene group optionally substituted by one or two methoxy groups; $R^c$ represents $C_{1-6}$ alkylene optionally substituted with a methyl or ethyl group and $R^d$ and $R^e$ independently represent $C_{1-6}$ alkyl; $R^f$ represents $C_{1-6}$ alkyl; $R^g$ represents hydrogen or phenyl optionally substituted by up to three groups selected from halogen, $C_{1-6}$ alkyl, or $C_{1-6}$ alkoxy; and Q is oxygen or NH.

Examples of suitable pharmaceutically acceptable in vivo hydrolysable ester groups include, for example, acyloxyalkyl groups such as acetoxymethyl, pivaloyloxymethyl, α-acetoxyethyl α-pivaloyloxyethyl, 1-(cyclohexylcarbonyloxy)prop-1-yl, and (1-aminoethyl)carbonyloxymethyl; alkoxycarbonyloxyalkyl groups,

such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula:

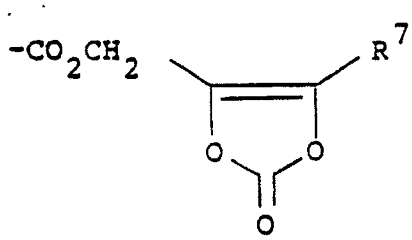

wherein $R^7$ is hydrogen, $C_{1-6}$ alkyl or phenyl.

Suitable pharmaceutically acceptable salts of the carboxy group of the compound of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)amine, cycloalkylamines such as dicyclohexylamine, or with procaine, dibenzylamine, N,N-dibenzylethylenediamine, 1-ephenamine, N-methylmorpholine, N-ethylpiperidine, N-benzyl-β- phenethylamine, dehydroabietylamine, N,N′-bisdehydro-abietylamine, ethylenediamine, or bases of the pyridine type such as pyridine, collidine or quinoline, or other amines which have been used to form salts with known penicillins and cephalosporins. Other useful salts include the lithium salt and silver salt.

One particularly preferred sub-group within the present invention provides a compound of formula (II) or a pharmaceutically acceptable salt or in vivo hydrolysable ester thereof:

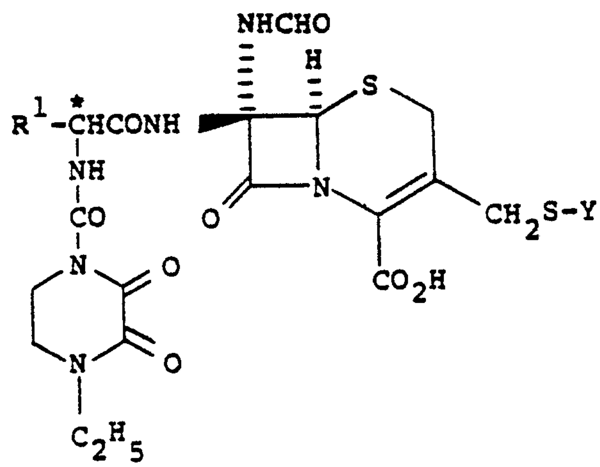

(II)

wherein $R^1$, * and Y are as defined with respect to formula (I) and $R^1$ is phenyl or substituted phenyl as hereinbefore defined.

Specific compounds within this invention include the following and pharmaceutically acceptable salts and in-vivo hydrolysable esters thereof:

3-[(1-Amino-1H-tetrazol-5-yl)thiomethyl]-7β-[D-2-(3,4-diacetoxyphenyl)-2-[(4-ethyl-2,3-dioxopiperazin- 1-yl)-carbonylamino]acetamido]-7α-formamidoceph-3-em-4-carboxylic acid;

3-[(1-Amino-1H-tetrazol-5-yl)thiomethyl]-7β-[D-2-(3,4-dihydroxyphenyl)-2-[(4-ethyl-2,3-dioxopiperazin-1-yl)-carbonylamino]acetamido]-7α-formamidoceph-3-em-4-carboxylic acid;

3-[(2-Chloroacetamido-1,3,4-thiadiazol-5-yl) thiomethyl]-7β-[D-2-[(4-ethyl-2,3-dioxopiperazin-1-yl) carbonylamino]-2-phenylacetamido]-7α-formamidoceph-3-em-4-carboxylic acid;

3-[(2-Amino-1,3,4-thiadiazol-5-yl)thiomethyl]-7β-[D-2-[(4-ethyl-2,3-dioxopiperazin-1-yl) carbonylamino]-2-phenylacetamido]-7α-formamidoceph-3-em-4-carboxylic acid;

3-[(2-Chloroacetamido-1,3,4-thiadiazol-5-yl)thiomethyl]-7β-[D,2-(3,4-dihydroxyphenyl)-2-[(4-ethyl-2,3-dioxopiperazin-1-yl)carbonylamino]acetamido]-7α-formamidoceph-3-em-4-carboxylic acid;

3-[(2-Amino-1,3,4-thiadiazol-5-yl)thiomethyl]-7β-[D-2-(3,4-dihydroxyphenyl)-2-[(4-ethyl-2,3-dioxopiperazin-1-yl)carbonylamino]acetamido]-7α-formamidoceph-3-em-4-carboxylic acid;

7β-[D-2-[(4-Ethyl-2,3-dioxopiperazin-1-yl)carbonylamino]-2-phenylacetamido]-7α-formamido-3-[(2-formamido-1,3,4-thiadiazol-5-yl)thiomethyl]ceph-3-em-4-carboxylic acid;

7β-[D-2-(3,4-Dihydroxyphenyl)-2-[(4-ethyl-2,3-dioxopiperazin-1-yl)carbonylamino]acetamido]-7α-formamido-3-[(2-formamido-1,3,4-thiadiazol-5-yl) thiomethyl]ceph-3-em-4-carboxylic acid;

3-[(1-Amino-1H-tetrazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylic acid;

7β-[D-2-[(4-Ethyl-2,3-dioxopiperazin-1-yl)carbonylamino]-2-phenylacetamido]-7α-formamido-3-[(1-isopropylidenamino-1H-tetrazol-5-yl)thiomethyl] ceph-3-em-4-carboxylic acid;

3-[(1-Amino-1H-tetrazol-5-yl)thiomethyl]-7β-[D-2-[(4-ethyl-2,3-dioxopiperazin-1-yl)carbonylamino]-2-(4-hydroxyphenyl)acetamido]-7α-formamidoceph-3-em-4-carboxylic acid;

7β-[D-2-(4-Acetoxyphenyl)-[2-(4-ethyl-2,3-dioxopiperazin-1-yl)carbonylamino]acetamido]-3-[(1-amino-1H-tetrazol-5-yl)thiomethyl]-7α-formamidoceph-3-em-4-carboxylic acid;

7β-[D-2-(3,4-Dihydroxyphenyl)-2-[(4-ethyl-2,3-dioxopiperazin-1-yl)carbonylamino]acetamido]-7α-formamido-3-[(1-methylamino-1H-tetrazol-5-yl) thiomethyl]ceph-3-em-4-carboxylic acid;

3-[[2-(3,4-Diacetoxybenzoylamino)-1,3,4-thiadiazol-5-yl]thiomethyl]-7β-[2-[(4-ethyl-2,3-dioxopiperazin-1-yl)-carbonylamino]-2-phenylacetamido]-7α-formamidoceph-3-em-4-carboxylic acid;

3-[(4-Amino-1,2,4-triazol-3-yl)thiomethyl]-7β-[D-2-(3,4-diacetoxyphenyl)-[2-(4-ethyl-2,3-dioxopiperaz in-1-yl)-carbonylamino]acetamido]-7α-formamidoceph-3-em-4-carboxylic acid; and

7β-[D-2-[(4-Ethyl-2,3-dioxopiperazin-1-yl)carbonylamino]-2-phenylacetamido]-3-[[2-(3,4-dihydroxybenzoylamino)-1,3,4-thiadiazol-5-yl]thiomethyl]-7α-formamidoceph-3-em-4-carboxylic acid; and

7β-[D-2-[(4-Ethyl-2,3-dioxopiperazin-1-yl)carbonylamino]-2-phenylacetamido]-7α-formamido-3-([2-[(5-hydroxy-4-oxo-1,4-dihydropyridin-2-yl)carbonylamino]-1,3,4-thiadiazol-5-yl]thiomethyl)ceph-3-em-4-carboxylic acid.

The antibiotic compounds according to the invention may be formulated for administration in any convenient way for use in human or veterinary medicine, according to techniques and procedures per se known in the art with reference to other antibiotics, and the invention therefore includes within its scope a pharmaceutical composition comprising an antibiotic compound according to the present invention such as, for example a pharmaceutically acceptable compound of formula (IA) or a salt or in vivo hydrolysable ester thereof above together with a pharmaceutically acceptable carrier or excipient. The compositions may be formulated for administration by any suitable route, such as oral or parenteral, or by topical application. The compositions may be in the form of tablets, capsules, powders, granules, lozenges, creams or liquid preparations, such as oral or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to

methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethyl-cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and if desired conventional flavouring or colouring agents. Suppositories will contain conventional suppository base, eg cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% to 99.5% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 mg to 12 g per day for an average adult patient (70 kg.), for instance 1500 mg per day, depending on the route and frequency of administration. Such dosages correspond to approximately 1.5 to 170 mg/kg per day. Suitably the dosage is from 1 to 6g. per day.

The daily dosage is suitably given by administering a compound of the invention several times in a 24-hour period. Typically, 250 mg. is administered 4 times a day although, in practice, the dosage and frequency of administration which will be most suitable for an individual patient will vary with the age, weight and response of the patients, and there will be occasions when the physician will choose a higher or lower dosage and a different frequency of administration. Such dosage regimens are within the scope of this invention.

No toxicological effects are indicated when a pharmaceutically acceptable compound of the invention of formula (IA) or a salt or in vivo hydrolysable ester thereof is administered in the above mentioned dosage range.

The antibiotic compounds according to the present invention may be the sole therapeutic agent in the compositions of the invention or a combination with other antibiotics and/or β-lactamase inhibitor may be employed.

Advantageously the compositions also comprise a compound of formula (III) or a pharmaceutically acceptable salt or ester thereof:

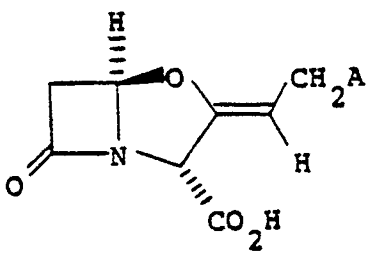

(III)

wherein A is hydroxyl; substituted hydroxyl; thiol; a group of formula $SO_2R^8$ wherein $R^8$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP O 053 893.

A further advantageous composition comprises a pharmaceutically acceptable antibiotic compound of the formula (IA) or a salt or in vivo hydrolysable ester thereof and a pharmaceutically acceptable carrier or excipient together with a β-lactamase inhibitor of formula (IV) or a pharmaceutically acceptable salt or in vivo hydrolysable ester thereof:

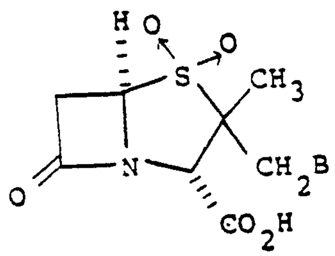

(IV)

wherein B is hydrogen, halogen or a group of formula:

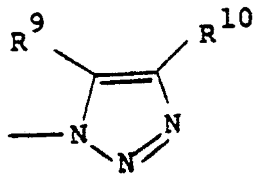

in which $R^9$ and $R^{10}$ are the same or different and each is hydrogen, $C_{1-6}$ alkoxycarbonyl, or carboxy or a pharmaceutically acceptable salt thereof.

Further suitable β-lactamase inhibitors include 6-alkylidene penems as described in European Patent Application No. 81301683.9 (Publication Number 0 041 768).

Further suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of a pharmaceutically acceptable antibiotic compound of this invention of the formula (IA) or a salt or in vivo hydrolysable ester thereof.

The pharmaceutically acceptable antibiotic compounds of the present invention of formula (IA) or salts or in vivo hydrolysable esters thereof are active against a broad range of Gram-positive and Gram-negative bacteria, and may be used to treat a wide range of bacterial infections including those in immunocompromised patients.

Amongst many other uses, the pharmaceutically acceptable compounds of the invention of formula (IA) or salts or in vivo hydrolysable esters thereof are of value in the treatment of respiratory tract and urinary tract infections in humans and may also be used to treat mastitis in cattle. A particular advantage of the antibacterially active compounds of this invention is their stability to β-lactamase enzymes and they are therefore effective against β-lactamase producing organisms.

The present invention further provides a process for the preparation of a compound of formula (I) which process comprises treating a compound of formula (V):

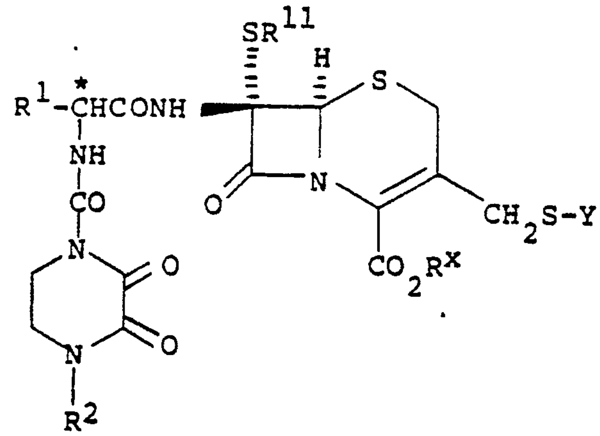

(V)

wherein Y, $R^1$, $R^2$, and * are as hereinbefore defined and wherein any reactive groups may be protected; $R^x$ is a readily removable carboxy protecting group; and $R^{11}$ is $C_{1-6}$ alkyl, aryl or benzyl; with a heavy metal ion such as mercury, silver, thallium, lead or copper; and thereafter in situ with a nucleophilic derivative of formamide as described in European Patent Application No. 84300338.5 (Publication Number 0 115 405).

At the end of the process described hereinabove and in other processes for the preparation of the compound of formula (I) described hereinbelow it may be necessary to remove protecting groups. Deprotection may be carried out by any convenient method known in the art that does not cause unwanted side reactions to occur to any appreciable extent. Methods that are particularly suitable for converting an acetoxy group in $R^1$ into a hydroxy group include treatment with aqueous sodium sulphite solution or aqueous sodium hydrogen carbonate solution, or treatment with an esterase, especially citrus acetylesterase. When a hydroxy group in $R^1$ is protected as a silyl ether, for example the trimethylsilyl ether, removal of the silyl group is normally carried out by acid hydrolysis.

Suitable groups for $R^x$ include ester derivatives of the carboxylic acid as described hereinabove for the group $R^3$. The derivative is preferably one which may readily be cleaved.

In an alternative aspect, the present invention provides a process for the preparation of a compound of formula (I) which process comprises treating a compound of formula (VI):

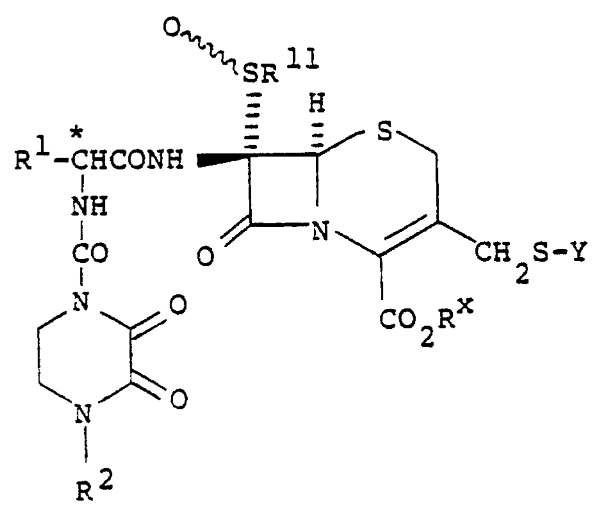

(VI)

wherein Y, $R^1$, $R^2$, *, $R^x$, and $R^{11}$ are as defined for formula (V) and wherein any reactive groups may be protected; with a nucleophilic derivative of formamide; and thereafter, if necessary, carrying out one or more of the following steps:

i) converting the group $R^x$ into a group $R^3$;

ii) removing any protecting groups; and

iii) converting the product into a salt.

The present invention further provides a process for the preparation of a compound of formula (I) which process comprises formylating a compound of formula (VII):

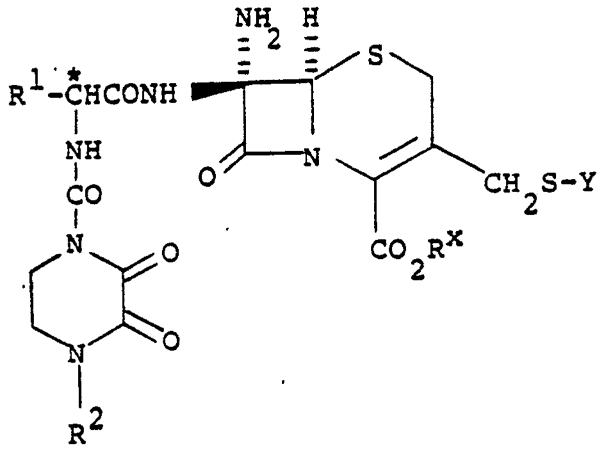

(VII)

wherein Y, $R^1$, $R^2$, $R^x$ and * are as hereinbefore defined and wherein any reactive groups may be protected; and thereafter, if necessary, carrying out one or more of the following steps:

i) converting the group $R^x$ into a group $R^3$;
ii) removing any protecting groups; or
iii) converting the product into a salt.

Suitable formylating agents include the reagent 4-formyl-2-methyl-1,3,4-thiadiazolin-5-thione (see H. Yazawa and S. Goto, Tetrahedron Letters, 1985, 26, 3703-3706), or mixed anhydrides such as formic acetic anhydride. The reaction may suitably be carried out in a temperature in the range -50° C to 30° C in aprotic solvent such as, for example, dichloromethane, chloroform, dimethylformamide, tetrahydrofuran, hexamethylphosphoramide, or dimethylsulphoxide, in the presence of a tertiary base. A preferred tertiary base employed in the reaction is a base of the pyridine type, such as pyridine, lutidine or picoline.

One process for preparing compounds within formula (VII) is disclosed in or is analogous to processes disclosed in European Patent Application No. 82303821.1 (Publication Number 0 071 395.

It will be apparent from the above that a process for preparing compounds of formula (I) comprises formamidylating a compound of formula (VIII):

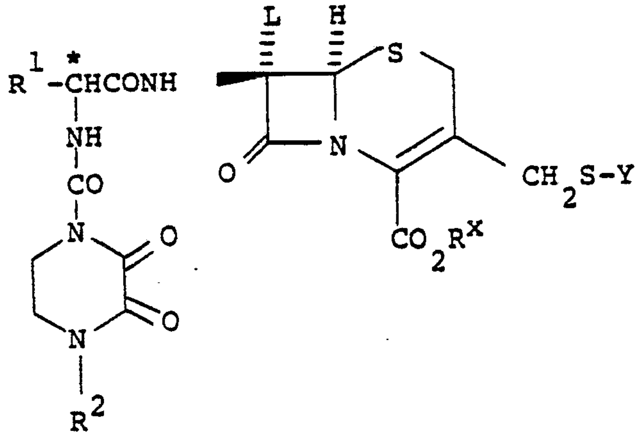

(VIII)

wherein L is $SR^{11}$, $SOR^{11}$ or $NH_2$; and Y, $R^1$, $R^2$, $R^{11}$, $R^x$ and * are as hereinbefore defined.

As used herein the term 'formamidylating' denotes converting the group L into the group -NHCHO.

The compounds of formula (I) may also suitably be prepared by reacting a compound of formula (IX) or

a salt thereof:

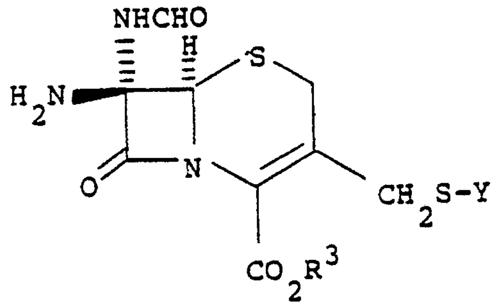

(IX)

wherein $R^3$ and Y are as hereinbefore defined; the 7β-amino group is optionally substituted with a group which permits acylation to take place; and any reactive groups may be protected; with an N-acylating derivative of an acid of formula (X):

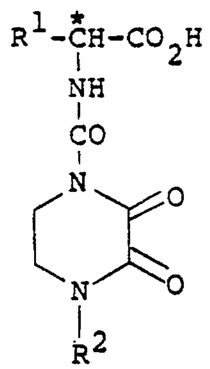

(X)

wherein $R^1$, $R^2$, and * are defined with respect to formula (I) and wherein any reactive groups may be protected; and thereafter, if necessary, carrying out one or more of the following steps:

i) converting a group $R^3$ into another group $R^3$;

ii) removing any protecting groups;

iii) converting the product into a salt.

Preferred compounds of the formula (IX) include salts and esters in which $R^3$ is as hereinbefore defined and in particular in which $R^3$ is diphenylmethyl or trimethylsilyl.

Suitable groups which permit acylation to take place and which are optionally present on the amino group of the starting material of the formula (IX) include silyl, stannyl and phosphorus groups, for example trialkylsilyl groups such as trimethylsilyl, trialkyltin groups such as tri-n-butyltin, groups of formula $-P.R^{12}R^{13}$ wherein $R^{12}$ is an alkyl, haloalkyl, aryl, aralkyl, alkoxy, haloalkoxy, aryloxy, aralkyloxy or dialkylamino group, $R^{12}$ is the same as $R^{13}$ or is halogen or $R^{12}$ and $R^{13}$ together form a ring; suitable such phosphorus groups being $-P(OC_2H_5)_2$, $-P(C_2H_5)_2$,

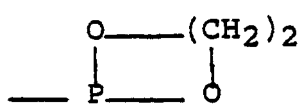

and

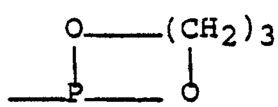

A group which may optionally be introduced onto the amino group in the compound of formula (IX) is trimethylsilyl.

An appropriate reactive N-acylating derivative of the acid (X) is employed in the above process.

Suitable N-acylating derivatives include an acid halide, preferably the acid chloride or bromide. Acylation with an acid halide may be effected in the presence of an acid binding agent, for example a tertiary amine (such as pyridine or dimethylaniline), molecular sieves, or an inorganic base (such as calcium

carbonate or sodium bicarbonate) or an oxirane, which binds hydrogen halide liberated in the acylation reaction. The oxirane is preferably a ($C_{1-6}$)-1,2-alkylene oxide - such as ethylene oxide or propylene oxide. The acylation reaction using an acid halide may be carried out at a temperature in the range -50°C to +50°C, preferably -20°C to +20°c, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof.

Alternatively, the reaction may be carried out in an unstable emulsion of water-immiscible solvent, especially an aliphatic ester or ketone, such as methyl isobutyl ketone or butyl acetate. Preferred solvents include tetrahydrofuran, and anhydrous chlorinated hydrocarbons, especially dichloromethane.

Acids of formula (X) may be prepared by the general method described in GB 1 508 064.

The acid halide may be prepared by reacting the acid (X) or a salt or suitable derivative thereof with a halogenating (eg chlorinating or brominating) agent such as phosphorus pentachloride, phosphorus oxychloride, thionyl chloride, oxalyl chloride, or phosgene.

Suitable derivatives of the acid (X) which may be employed in the above process include labile esters such as silyl esters. Suitable silyl esters include, for example, tri($C_{1-6}$)alkyl silyl esters, especially the trimethylsilyl ester.

Alternatively, the N-acylating derivative of the acid (X) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric, phosphorous, and phosphinic acids) or aromatic or aliphatic sulphonic acids (such as p-toluenesulphonic acid or methanesulphonic acid). When a symmetrical anhydride is employed, the reaction may be carried out in the presence of 2,6-lutidine as catalyst.

Alternative N-acylating derivatives of acid (X) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (VII) with an oxime.

Other reactive N-acylating derivatives of the acid (X) include the reactive intermediates formed by reaction in situ with a condensing agent such as a carbodiimide, for example, N,N′-diethyl-, di-n-propyl- or diisopropylcarbodiimide, N,N′-di-cyclohexylcarbodiimide, or N-ethyl-N′-[3-(dimethylamino)propyl]-carbodiimide; a suitable carbonyl compound, for example, N,N′-carbonyldiimidazole or N,N′-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

The compounds of formula (IX) herein which are, inter alia, intermediates for the compounds of formula (I) as hereinbefore defined may be prepared by reacting a compound of formula (XI) or acid addition salt thereof:

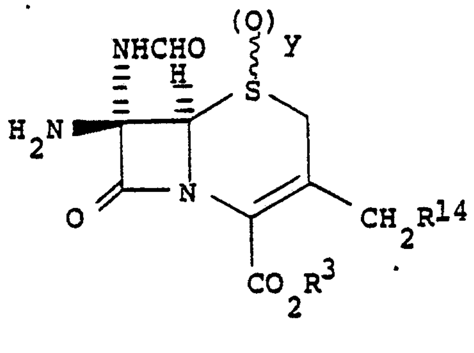

(XI)

wherein $R^3$ is as hereinbefore defined, $R^{14}$ is a leaving group and y is O or 1; with a thiol of the formula (XII):

Y-SH (XII)

wherein Y is as hereinbefore defined; with the proviso that when $R^{14}$ is an acyloxy group, the group $CO_2R^3$ must be in the free acid form or a salt thereof; and thereafter if necessary carrying out one or more of the following steps:

i) converting the group $R^3$ into a different group $R^3$;

ii) converting a sulphoxide (wherein y is 1) into a sulphide (wherein y is 0) by methods known in the art;

iii) converting the product into a salt.

Suitable leaving groups for $R^{14}$ include halogen such as iodine or bromine or an acyloxy group such as, for example, the acetoxy group.

Preferred groups for $R^{14}$ are acetoxy and bromo.

The thiol may be reacted as the free compound or a salt with an alkali metal such as sodium or potassium. This reaction is desirably conducted in a solvent. For example, use can be made of water, or organic solvents inert to the starting compounds, such as dimethylformamide, dimethylacetamide, dioxane, acetone, alcohol, dichloromethane, 1,2-dichloroethane, acetonitrile, dimethylsulfoxide or tetrahydrofuran, or mixtures thereof. The reaction temperature and time depend, among other factors, upon the starting compounds and solvent to be employed but generally the reaction is carried out at a selected temperature within the range of 0 to 100° C for a selected time of a few minutes to several days.

To prevent oxidation of the thiol it is advantageous to carry out the reaction in an inert gaseous atmosphere, e.g. argon gas.

The compounds of formula (I) may also suitably be prepared by reacting a compound of formula (XIII):

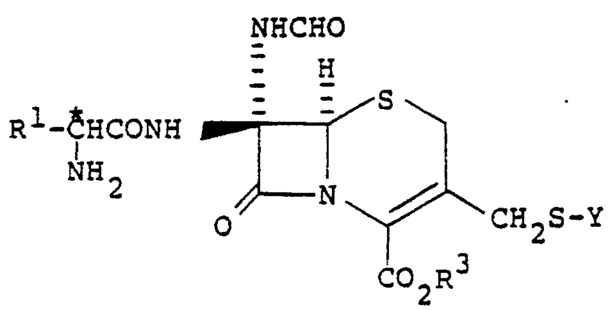

(XIII)

wherein $R^1$, $R^3$, Y, and * are as hereinbefore defined and the α-amino group in the 7β-side-chain is optionally substituted with a group which permits acylation to take place, and any reactive groups may be protected; with an N-acylating derivative of an acid of formula (XIV):

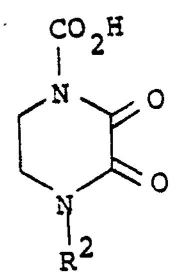

(XIV)

wherein $R^2$ is as hereinbefore defined and wherein any reactive groups may be protected; and thereafter, if necessary, carrying out one or more of the following steps:

i) converting the group $R^3$ into another group $R^3$;

ii) removing any protecting groups;

iii) converting the product into a salt.

The compounds of formula (XIII) herein which are inter alia intermediates for the compounds of formula (I) as hereinbefore defined may be prepared by reacting a compound of formula (IX) with an N-acylating derivative of an acid of formula (XV):

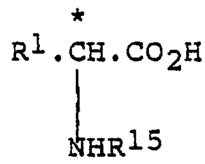

(XV)

wherein $R^1$ and * are as hereinbefore defined and $R^{15}$ is an amino-protecting group and thereafter removing protecting group $R^{15}$.

Suitable amino-protecting groups $R^{15}$ are those well-known in the art which may be removed under conventional conditions without disruption of the remainder of the molecule.

Examples of amino-protecting groups for $R^{15}$ include benzyl optionally substituted in the phenyl ring by one or two substituents selected from $C_{1-4}$ alkyl, $C_{1-4}$ alkoxy, trifluoromethyl, halogen or nitro; $C_{1-4}$ alkoxycarbonyl optionally substituted by a phenyl or biphenylyl group , for example tert-butoxycarbonyl or 1-methyl-1-(4-biphenylyl)ethoxycarbonyl; benzyloxycarbonyl optionally substituted as for benzyl above; allyloxycarbonyl; or trityl.

Compounds of formula (XIII) may also be prepared by reacting a compound of formula (IX) with an N-acylating derivative of an acid of formula (XVI):

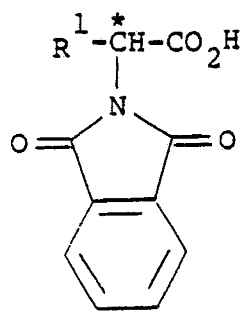

(XVI)

wherein $R^1$ and * are as hereinbefore defined; and thereafter converting the phthalimido group into an amino group by conventional methods.

Compounds of formula (XIII) may also be prepared by reacting a compound of formula (IX) with an N-acylating derivative of an α-azido acid of formula (XVII):

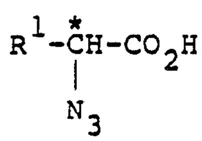

(XVII)

wherein $R^1$ and * are as hereinbefore defined; followed by conversion of the azido group into an amino group by conventional methods, for example by catalytic hydrogenation, or by reaction with triphenylphosphine followed by hydrolysis of the resultant phosphinimine.

In an alternative aspect, the resultant phosphinimine prepared as described above may be treated with an N-acylating derivative of an acid of formula (XIV) as hereinbefore defined to provide yet a further process for preparing compounds of formula (I). In this reaction the N-acylating derivative of the acid of formula (XIV) is preferably the acid chloride.

The compounds of the present invention of formula (I) may also suitably be prepared by reacting a compound of formula (XVIII):

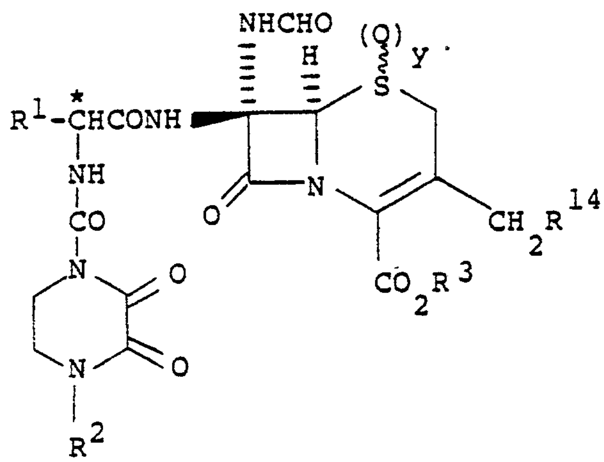

(XVIII)

wherein Y, $R^1$, $R^2$, $R^3$, $R^{14}$, y, and * are as defined hereinbefore, and wherein any reactive groups may be protected; with a thiol of formula (XII) with the proviso that when $R^{14}$ is an acyloxy group $-CO_2R^3$ must be in the free acid form or a salt thereof; and thereafter if necessary carrying out one or more of the following steps:

i) converting the group $R^3$ into a different group $R^3$;

ii) converting a sulphoxide (wherein y is 1) into a sulphide (wherein y is O) by methods known in the art;

iii) converting the product into a salt.

Suitable leaving groups $R^{14}$ include halogen such as iodide or bromide or an acyloxy groups such as, for example the acetoxy group.

The thiol (XII) may be reacted as the free compound or a salt with an alkali metal such as sodium or potassium under the same conditions as specified above for the preparation of compounds of formula (IX).

To prevent oxidation of the thiol it is advantageous to carry out the reaction in an inert gaseous atmosphere, e.g. argon gas.

The compounds of formula (IA) may be prepared by similar processes, to those described hereinabove as suitable for the preparation of a compound of the formula (I), except that each process for the preparation of the compound of formula (IA) further comprises, if necessary, the step of converting the product into a pharmaceutically acceptable salt or pharmaceutically acceptable in vivo hydrolysable ester.

It will be appreciated that compounds of formula (IX) and protected derivatives thereof are valuable intermediates, which are novel and form another preferred aspect of the present invention.

Specific compounds within formula (IX) include the following:

Sodium 7β-Amino-3-[(1-amino-1H-tetrazol-5-yl)thiomethyl]-7α-formamidoceph-3-em-4-carboxylate;

7β-Amino-3-[(1-amino-1H-tetrazol-5-yl)thiomethyl]-7α-formamidoceph-3-em-4-carboxylic acid;

Diphenylmethyl 7β-Amino-3-[(1-amino-1H-tetrazol-5-yl) thiomethyl]-7α-formamidoceph-3-em-4-carboxylate;

Diphenylmethyl 7β-Amino-7α-formamido-3-[(1-iso-propylideneamino-1H-tetrazol-5-yl)thiomethyl]ceph-3-em-4-carboxylate;

Sodium 7β-Amino-7α-formamido-3-[(1-methylamino-1H-tetrazol-5-yl)thiomethyl]ceph-3-em-4-carboxylate;

7β-Ammonio-7α-formamido-3-[(1-methylamino-1H-tetrazol-5-yl)thiomethyl]ceph-3-em-4-carboxylate;

Diphenylmethyl 7β-Amino-3-[(2-amino-1,3,4-thiadiazol-5-yl)thiomethyl]-7α-formamidoceph-3-em-4-carboxylate;

Diphenylmethyl 7β-Amino-3-[(2-chloroacetamido-1,3,4-thiadiazol-5-yl)thiomethyl]-7α-formamido ceph-3-em-4-carboxylate;

Sodium 7β-Amino-3-[(2-amino-1,3,4-thiadiazol-5-yl)thiomethyl]-7α-formamidoceph-3-em-4-carboxylate;

7β-Amino-3-[(2-amino-1,3,4-thiadiazol-5-yl) thiomethyl]-7α-formamidoceph-3-em-4-carboxylic acid;

Diphenylmethyl 7β-Amino-3-[(4-amino-1,2,4-triazol-3-yl)- thiomethyl]-7α-formamidoceph-3-em-4-carboxylate; and

Diphenylmethyl 7β-Amino-7α-formamido-3-[(1-formamido-1H-tetrazol-5-yl)thiomethyl]ceph-3-em-4-carboxylate.

The antibiotic compounds of the present invention are active against a wide range of Gram-negative and Gram-positive organisms including E.coli such as, for example ESS, JT4, JT425 and NCTC 10418; Pseudomonas Spp. such as Ps.aeruginosa for example 10662 and Dalgleish; Serratia marcescens US32; Klebsiella aerogenes A; Enterobacter cloacae N1; P.mirabilis such as, for example C977 and 889; P.morganii; P.rettgeri; B.subtilis; Staph. aureus such as, for example Oxford and Russell; N.catarrhalis 1502; Strep.faecalis I; β-Haemolytic Strep.CN10.

The following Examples illustrate the preparation and biological activity of the compounds of the present invention.

## Preparation 1

### 7β-Amino-7α-formamidocephalosporanic Acid

t-Butyl 7β-amino-7α-formamidocephalosporanate (10g, 27mmol) was dissolved in trifluoroacetic acid (100ml) at room temperature. After 1h the solution was evaporated and the residue triturated with ether to give the title product as an off-white solid which was filtered off and dried in vacuo (7.95g, 94%). This material was described previously for example in Example 34(e) of European Patent Application No. 0 071 395, as 7β-amino-7α-formamidocephalosporanic acid, trifluoroacetic acid salt.

## Example 1

### Sodium 3-[(1-Amino-1H-tetrazol-5-yl)thiomethyl]-7β-[D-2-(3,4-diacetoxyphenyl)-2-[(4-ethyl-2,3-dioxopiperazin-1-yl)carbonylamino]acetamido]-7α-formamidoceph-3-em-4-carboxylate

a) Sodium 7β-Amino-3-[(1-amino-1H-tetrazol-5-yl)thiomethyl]-7α-formamidoceph-3-em-4-carboxylate

To 7β-amino-7α-formamidocephalosporanic acid (460mg, 1.46mmol) in water (18ml) and tetrahydrofuran (6ml) was added saturated sodium hydrogen carbonate to pH 6.5. 1-Amino-5-mercapto-1H-tetrazole (151mg, 1.29mmol) (European Patent Application Publication No. 0 081 971) was added and the pH adjusted to 4.0 with 5N HCl. The resulting solution was heated at 60°C under argon for 6h, cooled, filtered and the filtrate concentrated to ca 1/3 volume. The concentrate was chromatographed on "Diaion HP20SS" resin, eluting with water. The product-containing fractions (HPLC monitoring) were combined, concentrated and the concentrate lyophilised to afford the title compound (400mg, 94%); $\lambda_{max}$ ($H_2O$) 264nm (ε 8670); $\nu_{max}$ (KBr) 3290, 3005, 1760, 1670, 1605, and 1515cm$^{-1}$; δ ($D_2O$) (major rotamer only) 3.40 and 3.70 (2H, ABq, J 17.6Hz), 4.01 and 4.25 (2H, ABq, J 13.4Hz), 5.11 (1H, s), and 8.11 (1H, s); [F.A.B. (+ve ion) (thioglycerol) $MH^+$ 395, $MNa^+$ 417].

b) 7β-Amino-3-[(1-amino-1H-tetrazol-5-yl)thiomethyl]-7α-formamidoceph-3-em-4-carboxylic Acid

Sodium 7β-amino-3-[(1-amino-1H-tetrazol-5-yl)thiomethyl]-7α-formamidoceph-3-em-4-carboxylate (277mg, 0.693mmol) was dissolved in water (10ml) and the pH adjusted to 2.0 with 5N-HCl. The resulting solution was lyophilised to give the title compound; δ [$(CD_3)_2SO$] inter alia (major rotamer only) 3.69 and 3.77 (2H, ABq, J 17.2Hz), 4.23 and 4.50 (2H, ABq, J 13.4Hz), 5.10 (1H, s), 7.00 (1H, br s, $D_2O$ exch.), 8.14 (1H, br s), and 10.01 (1H, br s, $D_2O$ exch.).

c) Diphenylmethyl 7β-Amino-3-[(1-amino-1H-tetrazol-5-yl)thiomethyl]-7α-formamidoceph-3-em-4-carboxylate

Method A

7β-Amino-7α-formamidocephalosporanic acid (1.55g, 4.9mmol) was dissolved in water (50ml) and THF (18ml) at pH 6.5, 1-amino-5-mercapto-1H-tetrazole (0.312g, 3.63mmol) added and the pH adjusted to 4.0. The reaction mixture was heated at 60° C for 6h under argon, cooled, filtered and the filtrate evaporated. The residue was re-dissolved in water (7ml), pH adjusted to 2.0 with 5N-HCl and the solvent evaporated. The residual foam was triturated in acetonitrile (20ml) and the resulting suspension treated with diphenyldiazomethane (1g, 5.15mmol) for 4h. The purple colour was then discharged by the addition of the minimum quantity of acetic acid and the mixture partitioned between ethyl acetate and water. The separated organic layer was washed with sodium hydrogen carbonate solution followed by brine, dried ($MgSO_4$) and evaporated. The residue was chromatographed on silica gel to give the title compound - (0.423g, 22%); $\nu_{max}$ ($CHCl_3$) 3200, 1780, 1710, and 1690cm$^{-1}$; δ [$(CD_3)_2CO$] (major rotamer only) 2.85 (2H, br s, exch), 3.63 and 3.75 (2H, ABq, J 17Hz), 4.26 and 4.52 (2H, ABq, J 13.4Hz), 5.22 (1H, s), 6.52 (2H, br s, $D_2O$ exch.), 6.96 (1H, s), 7.2-7.7 (10H, m), 8.10 (1H, br s, $D_2O$ exch.), and 8.26 (1H, d, J 1Hz, collapses to s on $D_2O$ exch); [FAB (+ve ion) (thioglycerol) $MH^+$ 539].

Method B

To 7β-amino-3-[(1-amino-1H-tetrazol-5-yl)thiomethyl]-7α-formamidoceph-3-em-4-carboxylic acid (250mg, 0.585mmol) suspended in acetonitrile (10ml) was added diphenyldiazomethane (250mg; 1.28mmol) and the reaction stirred at ambient temperature. The title product was isolated and purified as described in Method A.

d) Diphenylmethyl 3[-(1-Amino-1H-tetrazol-5-yl)thiomethyl]-7β-[D-2-(3,4-diacetoxyphenyl)-2-[4-ethyl-2,3-dioxopiperazin-1-yl)carbonylamino]acetamido]-7α-formamidoceph-3-em-4-carboxylate

Freshly prepared D-2-(3,4-diacetoxyphenyl)-2-[(4-ethyl-2,3-dioxopiperazin-1-yl)carbonylamino]acetyl chloride, generated by treatment of the corresponding acid (250mg, 0.575mmol) with oxalyl chloride (146mg; 1.15mmol) in dry dichloromethane containing N,N-dimethylformamide (½ drop) for 1.75h, was taken up in dry dichloromethane (2.6ml) and added to a stirred solution of diphenylmethyl 7β-amino-3-[(1-amino-1H-tetrazol-5-yl)thiomethyl]-7α-formamidoceph-3-em-4-carboxylate (206mg, 0.383mmol) and pyridine (30mg, 0.383mmol) in dry dichloromethane (5ml). After 1.5h the mixture was diluted with ethyl acetate (30ml) and washed with a little dilute sodium hydrogen carbonate solution. The separated organic phase was dried ($MgSO_4$) and evaporated to give a yellow residue which was chromatographed on silica gel to give the title compound (164mg, 45%) as a white solid; $\nu_{max}$ (Nujol) 3270, 1775, 1720, 1710, and 1690cm$^{-1}$; δ [$(CD_3)_2CO$] (major rotamer only) 1.17 (3H, t, J 7.2Hz), 2.25 and 2.26 (2 × 3H, each s), 3.07 and 3.33 (2H, ABq, J 16.2Hz), 3.50 (2H, q, J 7.2Hz), 3.6-3.75 (2H, m), 3.95-4.1 (2H, m), 4.27 and 4.64 (2H, ABq, J 13.3Hz), 5.26 (1H, s), 5.76 (1H, d, J 6.9Hz, collapses to s on $D_2O$ exch), 6.50 (2H, br s, $D_2O$ exch.), 6.94 (1H, s), 7.2-7.7 (14H, m), 8.30 (1H, d, J 1Hz, collapses to s on $D_2O$ exch), 8.53 (1H, br s, $D_2O$ exch), 8.82 (1H, br s, $D_2O$ exch), and 10.09 (1H, d, J 6.9Hz, exch); [F.A.B. (+ve ion) (thioglycerol) $MH^+$ 956].

e) Sodium 3-[(1-Amino-1H-tetrazol-5-yl)thiomethyl]-7β-[D-2-(3,4-diacetoxyphenyl)-2-[4-ethyl-2,3-dioxopiperazin-1-yl)carbonylamino]acetamido]-7α-formamidoceph-3-em-4-carboxylate

A solution of diphenylmethyl 3-[(1-amino-1H-tetrazol-5-yl)thiomethyl]-7β-[D-2-(3,4-diacetoxyphenyl)-2-[-(4- ethyl-2,3-dioxopiperazin-1-yl)carbonylamino]acetamido]-7α-formamidoceph-3-em-4-carboxylate (159mg, 0.166mmol) and anisole (539mg, 4.99mmol) in dry dichloromethane (5ml) at ice bath temperature was treated with trifluoroacetic acid (569mg, 4.99mmol). After 30 min, toluene (1ml) was added and the solution evaporated. The residue was triturated with dry ether to give a buff coloured solid which was collected by decantation of the ether. The solid was suspended in water (3ml), pH adjusted to 6.8, and the resulting solution passed through "Diaion HP20SS" resin, eluting first with water and then water-acetone mixture. The product-containing fractions (HPLC monitoring) were combined, concentrated and the concentrate lyophilised to give the title compound as an amorphous white solid (77mg, 59%); $\lambda_{max}$ ($H_2O$) 256nm (ε 12710); $\nu_{max}$ (KBr) 3410, 3295, 2980, 1770, 1710, 1680, 1610, and 1500cm$^{-1}$; δ ($D_2O$) inter alia (major rotamer only) 1.16 (3H, t, J 7.2Hz), 2.30 (6H, s), 2.94 and 3.35 (2H, ABq, J 17Hz), 3.48 (2H, q, J 7.2Hz), 3.6-3.75 (2H, m), 3.9-4.1 (3H, m), 4.31 (1H, d, J 15.5Hz), 5.22 (1H, s), 5.51 (1H, s), 7.29 (1H, d, J 8.4Hz), 7.40 (1H, d, J 2Hz), 7.50 (1H, dd, J 2 and 8.4Hz), and 8.11 (1H, s); [F.A.B. (+ve ion) (thioglycerol) $MH^+$ 812].

Example 2

Sodium 3-[(1-Amino-1H-tetrazol-5-yl)thiomethyl]-7β-[D-2-(3,4-dihydroxyphenyl)-2-[(4-ethyl-2,3-dioxopiperazin-1-yl)carbonylamino]acetamido]-7α-formamidoceph-3-em-4-carboxylate

To a stirred solution of sodium 3-[(1-amino-1H-tetrazol-5-yl)thiomethyl]-7β-[D-2-(3,4-diacetoxyphenyl)-2-[(4-ethyl-2,3-dioxopiperazin-1-yl)carbonylamino]acetamido]-7α-formamidoceph-3-em-4-carboxylate (115mg, 0.142mmol) in water (5ml) at pH 8.0 was added sodium sulphite (46mg, 0.365mmol) at room temperature. The pH was maintained at 9.0 (with 2.5% w/v solution of sodium carbonate) and progress of reaction monitored by HPLC. When all starting material was depleted, the pH was adjusted to 6.8 and the mixture chromatographed on "Diaion HP20SS" resin, eluting first with water and then water-tetrahydrofuran mixtures. The product-containing fractions (HPLC monitoring) were combined, concentrated, and the concentrate lyophilised to give the title compound as an amorphous white solid (75mg, 73%); $\lambda_{max}$ ($H_2O$) 260nm (ε 14040); $\nu_{max}$ (KBr) 3300, 2985, 1770, 1710, 1675, 1605, and 1515cm$^{-1}$; δ ($D_2O$) (major rotamer only) 1.16 (3H, t, J 7.3Hz), 3.05 (1H, d, J 16.9Hz), 3.35-3.55 [together 3H, m; q (J 7.3Hz) centred at 3.48 discernible], 3.55-3.75 (2H, m), 3.85-4.1 (together 3H, m), 4.27 (1H, d, J 13.5Hz), 5.22 (1H, s), 5.31 (1H, s), 6.8-7.0 (together 3H, m), and 8.10 (1H, s); [F.A.B. (+ve ion) (thioglycerol) $MH^+$ 728.].

Example 3

Sodium 3-[(1-Amino-1H-tetrazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylate

a) Diphenylmethyl 3-[(1-Amino-1H-tetrazol-5-yl)thiomethyl]-7β-[D-2-[(4-ethyl-2,3-dioxopiperazin-1-yl)-carbonylamino]-2-phenylacetamido]-7α-formamidoceph-3-em-4-carboxylate

Freshly prepared D-2-[(4-ethyl-2,3-dioxopiperazin-1-yl) carbonylamino]-2-phenylacetyl chloride, generated by treatment of the corresponding acid (23mg, 0.0725mmol with oxalyl chloride (18.4mg, 0.145mmol) in dry dichloromethane (2ml) containing N,N-dimethylformamide (1/10 drop) for 1.5h, was taken up in dry dichloromethane (1ml) and added to a stirred solution of diphenylmethyl 7β-amino-3-[(1-amino-1H-tetrazol-5-yl) thiomethyl]-7α-formamidoceph-3-em-4-carboxylate (26mg, 0.0483mmol) and pyridine (3.82g, 0.0483mmol) in dry dichloromethane (2ml). After 30 min the mixture was diluted with dichloromethane and washed with a little saturated aqueous sodium hydrogen carbonate solution. The separated organic phase was dried ($MgSO_4$) and evaporated to give a yellowish residue which was chromatographed on silica gel to give the title compound (10mg, 25%); $\nu_{max}$ ($CHCl_3$) 3260, 1785, 1715 and 1690cm$^{-1}$;

δ [$(CD_3)_2CO$] (major rotamer only) inter alia 1.18 (3H, t, J 7Hz), 3.22 and 3.35 (2H, ABq, J 16Hz), 3.52 (2H, q, J 7Hz), 3.65-3.8 (2H, m), 3.95-4.15 (2H, m), 4.33 and 4.55 (2H, ABq, J 13Hz), 5.27 (1H, s), 5.74 (1H, d, J 6Hz, collapses to s on $D_2O$ exch.), 6.55 (2H, br s, $D_2O$ exch.), 6.95 (1H, s), 7.2-7.7 (together 13H, m), 8.3 (1H, s), 8.56 and 8.73 (each 1H, br s, $D_2O$ exch.), and 10.04 (1H, d, J 6Hz, $D_2O$ exch.).

b) Sodium 3-[(1-Amino-1H-tetrazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylate

A solution of diphenylmethyl 3-[(1-amino-1H-tetrazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylate (10mg, 0.0119mmol) and anisole (38.6mg, 0.358mmol) in dry dichloromethane (3ml) at ice bath temperature was treated with trifluoroacetic acid (40.8mg, 0.358mmol). After 60 min, the solution was evaporated and the residue triturated with dry ether. The resulting solid was collected by decantation of ether, and suspended in water (2ml). The pH was adjusted to 7.0 to give a solution which was purified on a "Diaion HP20SS" column eluting first with water and then acetone-water mixtures. The product-containing fractions (HPLC monitoring) were combined, concentrated and the concentrate lyophilised to give the title compound as an amorphous white solid (3mg, 38%); $\nu_{max}$ (KBr) 3325, 1770, 1710, 1675, 1605, and 1505cm$^{-1}$; δ ($D_2O$; major rotamer only) 1.21 (3H, t, J 7Hz), 3.09 and 3.47 (2H, ABq, J 16Hz), 3.54 (2H, q, J 7Hz), 3.6-3.8 (together 2H, m), 3.85-4.1 (together 3H, m), 4.3 (1H, d, J 14Hz), 5.26 (1H, s), 5.54 (1H, s), 7.4-7.6 (together 5H, m), and 8.15 (1H, s).

Example 4

Sodium 7β-[D-2-[(4-Ethyl-2,3-dioxopiperazin-1-yl)carbonylamino]-2-phenylacetamido]-7α-formamido-3-[(1-isopropylideneamino-1H-tetrazol-5-yl)thiomethyl]-ceph-3-em-4-carboxylate

a) Diphenylmethyl 7β-Amino-7α-formamido-3-[(1-isopropylideneamino-1H-tetrazol-5-yl)thiomethyl]-ceph-3-em-4-carboxylate

To 7β-amino-7α-formamidocephalosporanic acid (215mg, 0.685mmol) in acetone (2ml) and water (6ml) at pH 6.5 was added a solution of 1-t-butyloxycarbonylamino-1H-tetrazol-5-thiol (149mg, 0.685mmol) (prepared from t-butyl carbazate using the method described in GB 2 117 767 A) in water (1.25ml) and acetone (1.25ml). The resulting solution was heated at 60°C under argon for 6h and then stored at ambient temperature for 12h. The pH was adjusted to 2.0 to give a yellow precipitate which was filtered off; the filtrate was treated with a little acetone giving more yellow precipitate, which was also filtered off. The solids were suspended in acetonitrile and treated overnight with excess diphenyldiazomethane.

The combined reaction mixtures were decolourised with glacial acetic acid, evaporated and the residue partitioned between ethyl acetate and dilute aqueous sodium hydrogen carbonate solution. The separated organic phase was dried ($MgSO_4$) and evaporated to give a syrup (0.51g) which was chromatographed on silica gel to afford the title compound (74mg, 27%); $\nu_{max}$ ($CHCl_3$) 3410, 1785, 1715, 1695, and 1625cm$^{-1}$; δ ($CDCl_3$; major rotamer only) 2.21 and 2.29 (each 3H, 2 × s), 2.40 (2H, br s, $D_2O$ exch.), 3.57 and 3.66 (2H, ABq, J 17Hz), 4.25 and 4.48 (2H, ABq, J 13.4Hz), 5.14 (1H, s), 6.69 (1H, br s, $D_2O$ exch.), 6.97 (1H, s), 7.2-7.7 (together 10H, m), and 8.17 (1H, s); [F.A.B. (+ve ion) (thioglycerol) MH+, 579, $MNa^+$ 601].

b) Diphenylmethyl 7β-[D-2-[(4-Ethyl-2,3-dioxopiperazin-1-yl)carbonylamino]-2-phenylacetamido]-7α-formamido-3-[(1-isopropylideneamino-1H-tetrazol-5-yl)thiomethyl]-ceph-3-em-4-carboxylate

Freshly prepared D-2-[(4-ethyl-2,3-dioxopiperazin-1-yl) carbonylamino]-2-phenylacetyl chloride, generated by treatment of the corresponding acid (36.4mg, 0.114mmol) with oxalyl chloride (29mg, 0.228mmol) in dry dichloromethane (2.5ml) containing N,N-dimethylformamide (1/10 drop) for 90 min, was taken up in dry dichloromethane (2ml) and added to a stirred solution of diphenylmethyl 7β-amino-7α-formamido-3-[(1-isopropylideneamino-1H-tetrazol-5-yl)thiomethyl]-ceph-3-em-4-carboxylate (44mg, 0.076mmol) and pyridine (6mg, 0.076mmol) in dry dichloromethane (3ml). After 15 min the mixture was diluted with dichloromethane

and washed with dilute sodium hydrogen carbonate solution, followed by dilute brine. The separated organic phase was dried ($MgSO_4$), evaporated and the residue chromatographed on silica gel to give the title compound (44mg, 67%); $\nu_{max}$ ($CHCl_3$) 3270, 1790, 1715, and 1690cm$^{-1}$; δ [$(CD_3)_2CO$; major rotamer only] 1.17 (3H, t, J 7Hz), 2.16 and 2.32 (each 3H, 2 × s), 3.20 and 3.36 (2H, ABq, J 16.5Hz), 3.50 (2H, q, J 7Hz), 3.60-3.75 (2H, m), 3.95-4.1 (2H, m), 4.33 and 4.59 (2H, ABq, J 13.5Hz), 5.28 (1H, s), 5.74 (1H, d, J 7Hz, collapses to s on $D_2O$ exch.), 6.94 (1H, s), 7.2-7.7 (15H, m), 8.28 (1H, d, J 0.8Hz, collapses to s on $D_2O$ exch.), 8.54 (1H, br s, $D_2O$ exch.), 8.76 (1H, br s, $D_2O$ exch.), 10.04 (1H, d, J 7Hz, $D_2O$ exch.); [F.A.B. (+ve ion) (thioglycerol) $MH^+$ 880].

c) Sodium 7β-[D-2-[(4-Ethyl-2,3-dioxopiperazin-1-yl)carbonylamino]-2-phenylacetamido]-7α-formamido-3-[(1-isopropylideneamino-1H-tetrazol-5-yl)thiomethyl]-ceph-3-em-4-carboxylate

A solution of diphenylmethyl 7β-[D-2-[(4-ethyl-2,3-dioxopiperazin-1-yl)carbonylamino]-2-phenylacetamido]-7α-formamido-3-[(1-isopropylideneamino-1H-tetrazol-5-yl)thiomethyl]-ceph-3-em-4-carboxylate (10mg, 0.0114mmol) and anisole (37mg, 0.342mmol) in dry dichloromethane (2ml) at ice bath temperature was treated with trifluoroacetic acid (39mg, 0.342mmol) for 15 min. The solution was evaporated, the residue dissolved in a little water and the pH adjusted to 7.0. The resulting cloudy solution was chromatographed on "Diaion HP20SS" resin, eluting with water and then acetone-water mixtures. The product-containing eluant (HPLC monitoring) was combined, concentrated and the concentrate lyophilised to afford the title compound as an amorphous white solid (5mg, 60%); $\nu_{max}$ (KBr) 3300, 2925, 1770, 1675, 1610, and 1505cm$^{-1}$; δ ($D_2O$; major rotamer only) 1.21 (3H, t, J 7Hz), 2.08 and 2.32 (each 3H, 2 × s), 3.08 and 3.47 (together 2H, ABq, J 17Hz), 3.54 (2H, q, J 7Hz), 3.6-3.8 (2H, m), 3.9-4.1 (2H, m), 4.07 and 4.32 (2H, ABq, J 13.4Hz), 5.27 (1H, s), 5.54 (1H, s), 7.4-7.6 (together 5H, m), and 8.15 (1H, s); [F.A.B. (+ve ion) (thioglycerol) $MH^+$ 736, $MNa^+$ 758].

## Example 5

Sodium 3-[(1-Amino-1H-tetrazol-5-yl)thiomethyl]-7β-[D-2-[(4-ethyl-2,3-dioxopiperazin-1-yl)carbonylamino]-2-(4-hydroxyphenyl)acetamido]-7α-formamidoceph-3-em-4-carboxylate

a) D-2-[(4-Ethyl-2,3-dioxopiperazin-1-yl)carbonylamino]-2-(4-trimethylsilyloxyphenyl)acetyl Chloride

A suspension of D-2-[(4-ethyl-2,3-dioxopiperazin-1-yl)carbonylamino]-2-(4-hydroxyphenyl) acetic acid (207mg, 0.618mmol) in dry dichloromethane (10ml), under argon, was reacted with N-methyl-N-trimethylsilyltrifluoroacetamide (492mg, 2.47mmol) with stirring at room temperature. After 4 hrs the mixture was evaporated at reduced pressure, the residue dissolved in dry dichloromethane (5ml) and reacted with thionyl chloride (88mg, 0.741mmol), monitoring by infra red spectroscopy. A further quantity of thionyl chloride (264mg, 2.22mmol) was added after 1hr and stirring continued for a further period of 18 hr. The mixture was evaporated at reduced pressure to give the product as a light yellow foam which was dissolved in dry dichloromethane (7ml); $\nu_{max}$ ($CH_2Cl_2$) 1790cm$^{-1}$.

b) Sodium 3-[(1-Amino-1H-tetrazol-5-yl-thiomethyl]-7β-[D-2-[(4-ethyl-2,3-dioxopiperazin-1-yl)carbonylamino]-2-(4-hydroxyphenyl)acetamido]-7α-formamidoceph-3-em-4-carboxylate

A suspension of 7β-amino-3-[(1-amino-1H-tetrazol-5-yl)thiomethyl]-7α-formamidoceph-3-em-4-carboxylic acid (107mg, 0.288mmol) in dry dichloromethane (5.6ml), under argon was reacted with N,O-bistrimethylsilylacetamide (70mg, 0.345mmol) at ambient temperature for 1hr. More N,O-bistrimethylsilylacetamide (70mg, 0.345mmol) was then added and stirring continued for a further 18 hrs which resulted in dissolution of most of the starting solid. This mixture was treated over 30 min with the solution prepared in part (a). After a further 30 min, water (10ml) was added, the mixture stirred vigorously (pH 2.0) for 20 min and then neutralised (pH 7.0) with saturated aqueous sodium hydrogen carbonate solution. The separated aqueous phase was chromatographed on a "Diaion HP20SS" column to afford the title compound (44mg, 21.5%) as

an amorphous white solid; $\lambda_{max}$ ($H_2O$) 224 ($\epsilon$ 19800) and 264nm (12050); $\nu_{max}$ (KBr) 3295, 2980, 1770, 1710, 1675, 1610, and 1510cm$^{-1}$; $\delta$ ($D_2O$; major rotamer only) 1.15 (3H, t, J 7.1Hz), 3.05 and 3.43 (2H, ABq, J 16Hz), 3.47 (2H, q, J 7.1Hz), 3.6-3.7 (2H, m), 3.9-4.05 (3H, m), 4.26 (1H, d, J 13.5Hz), 6.8-6.9 (2H, m), 7.3-7.4 (2H, m), and 8.08 (1H, s); [F.A.B. (+ve ion) (thioglycerol) $MH^+$ 712, $MNa^+$ 734].

Example 6

Sodium 7β-[D-2-(4-Acetoxyphenyl)-2-[(4-ethyl-2,3-dioxopiperazin-1-yl)carbonylamino]acetamido]-3-[(1-amino-1H-tetrazol-5-yl)thiomethyl]-7α-formamidoceph-3-em-4-carboxylate

a) Diphenylmethyl 7β-[D-2-(4-Acetoxyphenyl)-2-[(4-ethyl-2,3-dioxopiperazin-1-yl)carbonylamino]acetamido]-3-[(1-amino-1H-tetrazol-5-yl)thiomethyl]-7α-formamidoceph-3-em-4-carboxylate

Freshly prepared D-2-(4-acetoxyphenyl)-2-[(4-ethyl-2,3-dioxopiperazin-1-yl)carbonylamino]acetyl chloride, generated by treatment of the corresponding acid (187mg, 0.496mmol) with oxalyl chloride (126mg, 0.992mmol) in dry dichloromethane (2.5ml) containing N,N-dimethylformamide (1/5 drop) for 1.5h, was taken up in dry tetrahydrofuran (2.42ml) and added to a stirred solution of diphenylmethyl 7β-amino-3-[(1-amino-1H-tetrazol-5-yl)thiomethyl]-7α-formamidoceph-3-em-4-carboxylate (106mg, 0.197mmol) and pyridine (15.5mg, 0.197mmol) in dry tetrahydrofuran (1ml). After 25 min, the solution was diluted with ethyl acetate (40ml), transferred to a separating funnel and washed with dilute aqueous sodium hydrogen carbonate solution. The separated aqueous phase was extracted with ethyl acetate (20ml), the organic phases then combined, dried ($MgSO_4$) and evaporated. The resulting yellowish residue was chromatographed on silica gel. The first-eluted fraction was identical (t.l.c.) with the starting amine (14mg, 13%). The second-eluted fraction was the title compound (78mg, 44%); $\delta$ [$(CD_3)_2CO$; major rotamer only] 1.17 (3H, t, J 7.1Hz), 2.25 (3H, s), 3.14 and 3.35 (2H, ABq, J 16.3Hz), 3.50 (2H, q, J 7.1Hz), 3.6-3.8 (2H, m), 3.95-4.10 (2H, m), 4.29 and 4.58 (2H, ABq, J 13.4Hz), 5.26 (1H, s), 5.74 (1H, d, J 7Hz, collapses to s on $D_2O$ exch.), 6.52 (2H, br s, $D_2O$ exch.), 6.94 (1H, s), 7.05-7.75 (together 14H, m), 8.30 (1H, d, J 1Hz, collapses to s on $D_2O$ exch.), 8.55 (1H, br s, $D_2O$ exch.), 8.67 (1H, br s, $D_2O$ exch.), and 10.06 (1H, d, J 7Hz, $D_2O$ exch.).

b) Sodium 7β-[D-2-(4-Acetoxyphenyl)-2-[(4-ethyl-2,3-dioxopiperazin-1-yl)carbonylamino]acetamido]-3-[(1-amino-1H-tetrazol-5-yl)thiomethyl]-7α-formamidoceph-3-em-4-carboxylate

A solution of diphenylmethyl 7β-[D-2-(4-acetoxyphenyl)-2-[(4-ethyl-2,3-dioxopiperazin-1-yl)-carbonylamino]acetamido]-3-[(1-amino-1H-tetrazol-5-yl)thiomethyl]-7α-formamidoceph-3-em-4-carboxylate (72mg, 0.080mmol) and anisole (260mg, 2.41mmol) in dry dichloromethane (5ml) at ice bath temperature was treated with trifluoroacetic acid (275mg, 2.41mmol). After 45 min, the solution was evaporated and the residue triturated with dry ether. The resulting solid, obtained by decantation of ether was suspended in water (3ml), and pH adjusted to 7.0. The resulting solution was purified on a column packed with "Diaion HP20SS" resin, eluting first with water and then water-acetone mixtures. The product-containing fractions (HPLC monitoring) were combined, concentrated and the concentrate lyophilised to give the title product (30mg, 50%) as an amorphous white solid; $\lambda_{max}$ ($H_2O$) 258nm ($\epsilon$ 12,400); $\nu_{max}$ (KBr) 3315, 2980, 1765, 1710, 1675, 1610, and 1510cm$^{-1}$; $\delta$ ($D_2O$; major rotamer only) 1.18 (3H, t, J 7.1Hz), 2.31 (3H, s), 3.01 and 3.40 (2H, ABq, J 17Hz), 3.50 (2H, q, J 7.1Hz), 3.6-3.8 (2H, m), 3.9-4.1 (together 3H, m), 4.29 (1H, d, J 13.7Hz), 5.22 (1H, s), 5.52 (1H, s), 7.17 (2H, d, J 8.45Hz), 7.57 (2H, d, J 8.45Hz), and 8.11 (1H, s); [F.A.B. (+ve ion) (thioglycerol) $MH^+$, 754].

Example 7

Sodium 7β-[D-2-(3,4-Dihydroxyphenyl)-2-[(4-ethyl-2,3-dioxopiperazin-1-yl)carbonylamino]acetamido]-7α-formamido-3-[(1-methylamino-1H-tetrazol-5-yl)thiomethyl]ceph-3-em-4-carboxylate

a) Sodium 7β-Amino-7α-formamido-3-[(1-methylamino-1H-tetrazol-5-yl)thiomethyl]ceph-3-em-4-carboxylate

7β-Amino-7α-formamidocephalosporanic acid (374mg, 1.191mmol) was suspended in water (6ml) and pH adjusted to 7.0 with saturated aqueous sodium hydrogen carbonate solution. A solution of 1-methylamino-5-mercapto-1H-tetrazole (120mg, 0.916mmol) (European Patent Application Publication No. 0 081 971) in THF (2ml) was added to the above solution, and the resulting mixture heated at 60° C under argon for 6h. After a further 16h at 25° C, the mixture was filtered, pH adjusted to 6.8 and the filtrate concentrated. The concentrate was chromatographed on "Diaion HP20SS" resin, eluting with water. The product-containing fractions were combined, concentrated and the concentrate lyophilised to afford the title compound (280mg, 61%); $\lambda_{max}$ ($H_2O$) 266nm (ε 10,900); $\nu_{max}$ (KBr) 3270, 2995, 1755, 1675, 1605, and 1515cm$^{-1}$; δ ($D_2O$; major rotamer only) 2.94 (3H, s), 3.42 and 3.74 (2H, ABq, J 17.5Hz), 4.05 and 4.31 (2H, ABq, J 13.4Hz), 5.14 (1H, s), and 8.14 (1H, s); [F.A.B. (+ve ion) (thioglycerol) $MH^+$ 409, $MNa^+$ 431].

b) 7β-Ammonio-7α-formamido-3-[(1-methylamino-1H-tetrazol-5-yl)thiomethyl]ceph-3-em-4-carboxylate

A solution of sodium 7β-amino-7α-formamido-3-[(1-methylamino-1H-tetrazol-5-yl)thiomethyl]ceph-3-em-4-carboxylate (280mg, 0.727mmol) in water (2ml) was adjusted to pH 2.0 with 5N-HCl. The resulting gelatinous solid was treated with a little acetone and then filtered to give the product as an off-white amorphous solid (43mg, 11%); δ [$(CD_3)_2SO$; major rotamer only] 2.81 (3H, d, J 5.3Hz, collapses to s on $D_2O$ exch.), 3.40 (3H, br s, $D_2O$ exch.), 3.49 and 3.73 (2H, ABq, J 17.5Hz), 4.16 and 4.45 (2H, ABq, J 13.3Hz), 5.01 (1H, s), 7.38 (1H, q, J 5.3Hz, $D_2O$ exch.), 8.04 (1H, s), and 9.11 (1H, s, $D_2O$ exch.).

Concentration and lyophilisation of the filtrate from above gave a white solid (195mg) which possessed identical NMR characteristics to those described above.

c) Sodium 7β-[D-2-(3,4-Dihydroxyphenyl)-2-[(4-ethyl-2,3-dioxopiperazin-1-yl)carbonylamino]acetamido]-7α-formamido-3-[(1-methylamino-1H-tetrazol-5-yl)thiomethyl]ceph-3-em-4-carboxylate

i) A suspension of D-2-(3,4-dihydroxyphenyl)-2-[(4-ethyl-2,3-dioxopiperazin-1-yl)carbonylamino]acetic acid (948mg, 2.569mmol) in dry dichloromethane (60ml), under argon, was reacted with N-methyl-N-trimethylsilyltrifluoroacetamide (4.09g, 20.6mmol) and chlorotrimethylsilane (223mg, 2.055mmol) for 18hrs and then evaporated. The residue was redissolved in dry dichloromethane (25ml) and reacted with thionyl chloride (611mg, 5.138mmol) for 2hrs and the mixture evaporated at reduced pressue. The residue was dissolved in dry tetrahydrofuran (10ml).

ii) To a suspension of 7β-ammonio-7α-formamido-3-[(1-methylamino-1H-tetrazol-5-yl)thiomethyl]ceph-3-em-4-carboxylate (88mg, 0.228mmol) in dry tetrahydrofuran (5ml) under argon was treated with N-methyl-N-trimethylsilyltrifluoroacetamide (MSTFA) (159mg) 0.798mmol) in one portion and the mixture stirred for 90 min. More MSTFA (159mg) was then added, the mixture heated under reflux 30 min and then cooled to ambient temperature. The resulting mixture was treated with a portion of the acid chloride solution prepared in part (i) (1.5ml, containing 0.385mmol) for 15h at ambient temperature. Water (5ml) was added and the solution stirred vigorously for 20 min and the pH adjusted to 6.5 with dilute aqueous sodium hydrogen carbonate solution. The mixture was filtered and the filtrate concentrated to ca. 4ml. The concentrate was purified on a "Diaion HP20SS" column, eluting first with water and then acetone-water mixtures. The product-containing fractions were combined, concentrated and the concentrate lyophilised to afford the title compound (20mg, 11%) as a light yellow solid; $\lambda_{max}$ ($H_2O$) 270nm (ε 11,250); $\nu_{max}$ (KBr) 3285, 2980, 1770, 1675, 1610, and 1505cm$^{-1}$; δ ($D_2O$; major rotamer only) 1.16 (3H, t, J 7.2Hz), 2.88 (3H, s), 3.04 (1H, d, J 17.2Hz), 3.3-3.6 (together 3H, m), 3.6-3.8 (together 2H, m), 3.9-4.1 (together 3H, m), 4.29 (1H, d, J 13.4Hz), 5.21 (1H, s), 5.29 (1H, s), 6.8-7.0 (together 5H, m), and 8.10 (1H, s); [F.A.B. (+ve ion) (thioglycerol) $MH^+$ 742, $MNa^+$ 764].

Example 8

Sodium 3-[(2-Chloroacetamido-1,3,4-thiadiazol-5-yl) thiomethyl]-7β-[D,2-[(4-ethyl-2,3-dioxopiperazin-1-yl)-carbonylamino]-2-phenylacetamido]-7α-formamidoceph-3-em-4-carboxylate

Method A

(a) Diphenylmethyl 7β-Amino-3-[(2-amino-1,3,4-thiadiazol-5-yl)thiomethyl]-7α-formamidoceph-3-em-4-carboxylate

To 7β-amino-7α-formamidocephalosporanic acid (200mg, 0.635mmol) in water (5ml) and tetrahydrofuran (1ml) was added saturated sodium hydrogen carbonate to pH 6.5. 5-Amino-2-mercapto-1,3,4-thiadiazole (74mg, 0.56mmol) was added and the pH adjusted to 4.0 with 5N HCl. The resulting solution was heated at 60°C under argon for 6h, cooled and the pH adjusted to 2.0 with 5N HCl. The mixture was concentrated, dried in vacuo for 1h and then diphenyldiazomethane was added to the residue suspended in acetonitrile (10ml). After stirring for 20h, glacial acetic acid was added and the mixture evaporated. The residue was taken up in ethyl acetate and washed successively with water, dilute aqueous sodium hydrogen carbonate, brine, dried ($MgSO_4$) and evaporated. Chromatography on silica gel afforded the title product (69mg, 20%); $\nu_{max}$ (KBr) 3300, 1770, 1720, 1675, and 1610cm$^{-1}$; $\delta_H$ [$(CD_3)_2CO$] (major rotamer only) 2.76 (2H, s, exch.), 3.60 and 3.68 (2H, ABq, J 16.6Hz), 4.12 and 4.38 (2H, ABq, J 13.2Hz), 5.25 (1H, s), 6.68 (2H, br s, exch.), 6.90 (1H, s), 7.2-7.7 (10H, m), 8.13 (1H, s, exch.), and 8.25 (1H, d, J 1.3Hz). [F.A.B. (+ve ion) (thioglycerol) $MH^+$ 555].

b) Diphenylmethyl 7β-Amino-3-[(2-chloroacetamido-1,3,4-thiadiazol-5-yl)thiomethyl]-7α-formamido ceph-3-em-4-carboxylate

To diphenylmethyl 7β-amino-3-[(2-amino-1,3,4-thiadiazol-5-yl)thiomethyl]-7α-formamidoceph-3-em-4-carboxylate (66mg, 0.12mmol) in dry THF (2ml) at 0°C was added pyridine (10.2mg, 0.13mmol) in dry THF (0.4ml) followed by chloroacetyl chloride (12.1mg, 0.11mmol) in dry THF (0.4ml). After 15 min the reaction mixture was diluted with ethyl acetate and washed successively with very dilute hydrochloric acid, dilute sodium hydrogen carbonate, brine, dried ($MgSO_4$) and evaporated. Chromatography on silica gel gave the title product (44mg, 60%); $\nu_{max}$ ($CHCl_3$) 3350, 1780, 1720 and 1690cm$^{-1}$; $\delta_H$ [$(CD_3)_2CO$] (major rotamer) 3.62 and 3.73 (2H, ABq, J 16.5Hz), 4.22 and 4.56 (2H, ABq, J 13.2Hz), 4.48 (2H, s), 5.25 (1H, s), 6.91 (1H, s), 7.2-7.8 (11H, m), 8.09 (1H, s, exch.), and 8.25 (1H, d, J 1.1Hz).

c) Diphenylmethyl 3-[(2-Chloroacetamido-1,3,4-thiadiazol-5-yl)thiomethyl]-7β-[D,2-[(4-ethyl-2,3-dioxopiperazin-1-yl)carbonylamino]-2-phenylacetamido]-7α-formamidoceph-3-em-4-carboxylate

Diphenylmethyl 7β-amino-3-[(2-chloroacetamido-1,3,4-thiadiazol-5-yl)thiomethyl]-7α-formamidoceph-3-em-4-carboxylate (44mg, 0.07mmol) was dissolved in dry dichloromethane (2ml) at 0°C and freshly prepared D-2-[(4-ethyl-2,3-dioxopiperazin-1-yl)carbonylamino]phenylacetyl chloride (generated by treatment of the corresponding acid (33mg, 0.10mmol) with oxalyl chloride (28mg, 0.22mmol) in dry dichloromethane containing N,N-dimethylformamide (cat.) for 1.25h) in dichloromethane (1ml) added, followed by pyridine (6mg, 0.08mmol) in dichloromethane (0.2ml). After 30 min the reaction was diluted with ethyl acetate and washed successively with dilute hydrochloric acid, dilute aqueous sodium hydrogen carbonate, brine, dried ($MgSO_4$) and evaporated. The residue was chromatographed on silica gel to provide the title product (38mg, 58%); $\nu_{max}$ ($CHCl_3$) 3280, 1780, 1715 and 1690cm$^{-1}$; $\delta_H$ [$(CD_3)_2CO$] (major rotamer) 1.17 (3H, t, J 7.1Hz), 3.19 and 3.28 (2H, ABq, J 16Hz), 3.50 (2H, m), 3.6-3.8 (2H, m), 4.0-4.1 (2H, m), 4.29 and 4.58 (2H, ABq, J 13.3Hz), 4.47 (2H, s), 5.28 (1H, s), 5.73 (1H, d, J 7Hz), 6.87 (1H, s), 7.2-7.7 (16H, m), 8.28 (1H, d, J 1Hz), 8.56 (1H, s, exch.), 8.69 (1H, s, exch.) and 10.0 (1H, d, J 7Hz, exch.). [F.A.B. (+ve ion) (thioglycerol) $MH^+$ 932].

d) Sodium 3-[(2-Chloroacetamido-1,3,4-thiadiazol-5-yl)thiomethyl]-7β-[D,2-[(4-ethyl-2,3-dioxopiperazin-1-yl)-carbonylamino]-2-phenylacetamido]-7α-formamidoceph-3-em-4-carboxylate

To a solution of diphenylmethyl 3-[(2-chloroacetamido-1,3,4-thiadiazol-5-yl)thiomethyl]-7β-[D,2-[(4-ethyl-2,3-dioxopiperazin-1-yl)carbonylamino]-2-phenylacetamido]-7α-formamidoceph-3-em-4-carboxylate (35mg, 0.038mmol) in dry dichloromethane (3ml) at 0°C was added anisole (0.14ml, 1.29mmol) and trifluoroacetic acid (0.16ml, 2.07mmol). After 30 min the solution was evaporated and then re-evaporated from toluene;

this was repeated (× 2). The residue was dried in vacuo for 30 min, triturated with ether and the solid taken up in water at pH 6.5 and chromatographed on 'Diaion' HP20SS. The product containing fractions were lyophilised to give the title product (6mg, 20%); $\nu_{max}$ (KBr) 3420, 1770, 1705, 1675, and 1605cm$^{-1}$; $\delta_H$ ($D_2O$) (major rotamer) 1.16 (3H, t, J 7.2Hz), 2.98 and 3.33 (2H, ABq, J 16.8Hz), 3.4-3.6 (2H, m), 3.6-3.8 (2H, m), 3.88 and 4.21 (2H, ABq, J 13.6Hz), 3.9-4.1 (2H, m), 4.35 (2H, s), 5.25 (1H, s), 5.51 (1H, s), 7.3-7.6 (5H, m), and 8.10 (1H, s). [F.A.B. (+ve ion) (thioglycerol) $MH^+$ 788].

Method B

e) Sodium 7β-Amino-3-[(2-amino-1,3,4-thiadiazol-5-yl)thiomethyl]-7α-formamidoceph-3-em-4-carboxylate

To 7β-amino-7α-formamidocephalosporanic acid (200mg, 0.635mmol) in water (5ml) and tetrahydrofuran (1.7ml) was added saturated sodium hydrogen carbonate to pH 6.5. 5-Amino-2-mercapto-1,3,4-thiadiazole (74mg, 0.56mmol) was added, the pH adjusted to 4.0 with 5N HCl and the mixture heated at 60° C under argon for 6h. The cooled solution was adjusted to pH 6.5 with saturated sodium hydrogen carbonate, concentrated and the residue chromatographed on 'Diaion' HP20SS to afford the title product - (108mg, 31%); $\nu_{max}$ (KBr) 3360, 1755, 1670, and 1603cm$^{-1}$; $\delta_H$ ($D_2O$) (major rotamer) 3.34 and 3.74 (2H, ABq, J 17.4Hz), 3.72 and 4.26 (2H, ABq, J 13.8Hz), 5.12 (1H, s), and 8.13 (1H, s).

f) Sodium 3-[(2-Chloroacetamido-1,3,4-thiadiazol-5-yl)thiomethyl]-7β-[D,2-[(4-ethyl-2,3-dioxopiperazin-1-yl)-carbonylamino]-2-phenylacetamido]-7α-formamidoceph-3-em-4-carboxylate

Sodium 7β-amino-3-[(2-amino-1,3,4-thiadiazol-5-yl)thiomethyl]-7α-formamidoceph-3-em-4-carboxylate (250mg, 0.45mmol) in water (1.5ml) was acidified to pH 1.5 with 5N HCl. The mixture was cooled in ice/water for 30 min, the precipitate filtered off and washed with acetone. The acid product was dried in vacuo (169mg, 70%); $\nu_{max}$ (KBr) 3300, 1800, 1780, 1665 and 1615cm$^{-1}$; $\delta_H$ ($[CD_3]_2SO$) (major rotamer) 3.35 (3H, br s, exch.), 3.39 and 3.64 (2H, ABq, J 17.2Hz), 4.01 and 4.21 (2H, ABq, J 13.1Hz), 5.01 (1H, s), 7.36 (2H, s, exch.), 8.04 (1H, s), and 9.13 (1H, s, exch.). The acid product (100mg) was suspended in dry tetrahydrofuran (10ml) containing N,O-bis(trimethylsilyl)acetamide (0.195mg, 0.9mmol) and trimethylsilyl chloride (7mg, 0.06mmol) and heated under argon at reflux for 15 min. The resulting solution was cooled to 0° C and chloroacetyl chloride (28mg, 0.25mmol) in tetrahydrofuran (2ml) added dropwise, followed after 30 min by freshly prepared D-2-[(4-ethyl-2,3-dioxopiperazin-1-yl)carbonylamino]phenylacetyl chloride (generated by treatment of the corresponding acid (75mg, 0.25mmol) with oxalyl chloride (64mg, 0.5mmol) in dry dichloromethane containing N,N-dimethylformamide (cat.) for 1.25h) in tetrahydrofuran (2ml) dropwise. After warming to room temperature over 3h, the reaction mixture was diluted with water, the pH adjusted to 6.5 with saturated sodium hydrogencarbonate and concentrated in vacuo. The concentrate was chromatographed on 'Diaion' HP20SS resin and the product containing fractions lyophilised to give the title product (58mg, 31%), as described in Example 8(d).

Example 9

Sodium 3-[(2-Amino-1,3,4-thiadiazol-5-yl)thiomethyl]-7β-[D-2-[(4-ethyl-2,3-dioxopiperazin-1-yl)-carbonylamino]-2-phenylacetamido]-7α-formamidoceph-3-em-4-carboxylate

To a solution of sodium 3-[(2-chloroacetamido-1,3,4-thiadiazol-5-yl)thiomethyl]-7β-[D,2-[(4-ethyl-2,3-dioxopiperazin-1-yl)carbonylamino]-2-phenylacetamido]-7α-formamidoceph-3-em-4-carboxylate (50mg, 0.064mmol) in water (3ml) was added sodium N-methyldithiocarbamate (16mg, 0.128mmol) and after 4h the reaction mixture was poured onto 'Diaion' HP20SS resin. Elution with water, then acetone/water mixtures afforded after lyophilisation of the product-containing fractions, the title compound (24mg, 53%); $\lambda_{max}$ ($H_2O$) 260nm (ε 13,600); $\nu_{max}$ (KBr) 3300, 1770, 1710, 1690, 1675, and 1610cm$^{-1}$; $\delta_H$ ($D_2O$) (major rotamer only) 1.22 (3H, t, J 7.1Hz), 2.98 and 3.39 (2H, ABq, J 16.9Hz), 3.45-3.60 (2H, m), 3.60-3.80 (2H, m, obscures 1H, d), 3.9-4.1 (2H, m), 4.32 (1H, d, J 13.4Hz, lower field arm of ABq), 5.27 (1H, s), 5.54 (1H, s), 7.35-7.60 (5H, m) and 8.15 (1H, s). [F.A.B. (+ve ion) (thioglycerol) $MH^+$ 712].

Example 10

Sodium 7β-[D-2-[(4-Ethyl-2,3-dioxopiperazin-1-yl)carbonylamino]-2-phenylacetamido]-7α-formamido-3-[(2-formamido-1,3,4-thiadiazol-5-yl)thiomethyl]ceph-3-em-4-carboxylate

Sodium 3-[(2-amino-1,3,4-thiadiazol-5-yl)thiomethyl]-7β-[D-2-[(4-ethyl-2,3-dioxopiperazin-1-yl)-carbonylamino]-2-phenylacetamido]-7α-formamidoceph-3-em-4- carboxylate (10mg, 0.014mmol) in water (3ml) was acidified to pH 3 with dilute hydrochloric acid, the mixture lyophilised and the resulting material suspended in dichloromethane (10ml) containing N-methyl-N-(trimethylsilyl)trifluoroacetamide (28mg, 0.14mmol). After stirring for 3h under argon, further N-methyl-N-(trimethylsilyl)trifluoroacetamide (28mg, 0.14mmol) was added and the mixture refluxed for 30 min. To the resulting solution was added formic-acetic anhydride (30mg, 0.35mmol) at room temperature and after 1h, the solution was evaporated, the residue taken up in tetrahydrofuran/water, and the pH adjusted to 6.5 with saturated sodium hydrogencarbonate. The solution was then concentrated and the residue chromatographed on "Diaion" HP20SS resin to afford, after lyophilisation of the relevant fractions, the title product (3mg, 29%); $\nu_{max}$ (KBr) 3420, 1770, 1710, 1675, and 1610cm$^{-1}$; $\delta_H$ ($D_2O$) (major rotamer only) 1.17 (3H, t, J 7.3Hz), 2.95 and 3.31 (2H, ABq, J 16.7Hz), 3.45-3.60 (2H, m), 3.60-3.75 (2H, m), 3.9-4.1 (2H, m), 3.86 and 4.26 (2H, ABq, J 13.7Hz), 5.24 (1H, s), 5.50 (1H, s), 7.35-7.55 (5H, m), 8.10 (1H, s), and 8.42 (1H, s). [F.A.B. (+ve ion) (thioglycerol) $MH^+$ 740].

Example 11

Sodium 3-[[2-(3,4-Diacetoxybenzoylamino)-1,3,4-thiadiazol-5-yl]thiomethyl]-7β-[2-[(4-ethyl-2,3-dioxopiperazin-1-yl)carbonylamino]-2-phenylacetamido]-7α-formamidoceph-3-em-4-carboxylate

Sodium 3-[(2-amino-1,3,4-thiadiazol-5-yl)thiomethyl]-7β-[D-2-[(4-ethyl-2,3-dioxopiperazin-1-yl)-carbonylamino]-2-phenylacetamido]-7α-formamidoceph-3-em-4-carboxylate (40mg, 0.056mmol) in water (5ml) was acidified to pH 3.0 with dilute hydrochloric acid, the mixture lyophilised and the resulting acid suspended in dichloromethane (15ml) containing N-methyl-N-(trimethylsilyl)trifluoroacetamide (223mg, 1.12mmol) under argon and refluxed for 30 min. The resulting cooled solution was treated with diacetoxybenzoyl chloride (14mg, 0.056mmol) in dichloromethane (0.3ml) and stirred for 1h. The reaction mixture was evaporated, the residue redissolved in tetrahydrofuran/water and the pH adjusted to 6.5 with saturated sodium hydrogen carbonate. The title product (26mg, 50%) was isolated using the procedure described in Example 10; $\nu_{max}$ (KBr) 3400, 3300, 1770, 1710, 1675, and 1610cm$^{-1}$; $\delta_H$ ($D_2O$ - $[CD_3]_2CO$) (major rotamer) 1.23 (3H, t, J 7Hz), 2.40 (6H, s), 3.19 and 3.44 (2H, ABq, J 18Hz), 3.55 (2H, q, J 7Hz), 3.76 (2H, m), 4.07 (2H, m), 4.2-4.5 (2H, m), 5.31 (1H, s), 5.63 (1H, s), 7.3-7.6 (8H, m), and 8.18 (1H, s). [F.A.B. (+ve ion) (thioglycerol) $MH^+$ 910].

Example 12

Sodium 3-[(2-Chloroacetamido-1,3,4-thiadiazol-5-yl)thiomethyl]-7β-[D,2-(3,4-dihydroxyphenyl)-2-[(4-ethyl-2,3-dioxopiperazin-1-yl)carbonylamino]acetamido]-7α-formamidoceph-3-em-4-carboxylate

Sodium 7β-amino-3-[(2-amino-1,3,4-thiadiazol-5-yl)thiomethyl]-7α-formamidoceph-3-em-4-carboxylate (100mg, 0.18mmol) was acidified, silylated and sequentially ? ylated with chloroacetyl chloride (28mg, 0.25mmol) and D-2-[3,4-bis(trimethylsilyloxy)phenyl]-2-[(4-ethyl-2,3-dioxopiperazin-1-yl)carbonylamino]acetyl chloride (105mg, 0.23mmol) (prepared by the procedure described in Example 7(c)(i)) as described in Example 8f. The title product was then isolated as described in Example 8f; $\nu_{max}$ (KBr) 3300, 1770, 1710, 1675, and 1605cm$^{-1}$; $\delta_H$ ($D_2O$) (major rotamer) 1.16 (3H, t, J 7.1Hz), 3.05 (1H, d, J 16.8Hz, higher field arm of ABq), 3.3-3.55 (3H, m), 3.6-3.8 (2H, m), 3.8-4.1 (3H, m), 4.16 (1H, d, J 13.7Hz, lower field arm of ABq), 4.33 (2H, s), 5.27 (1H, s), 5.35 (1H, s), 6.8-7.3 (3H, m) and 8.10 (1H, s). [F.A.B. (+ve ion) (thioglycerol) $MH^+$ 820].

Example 13

Sodium 3-[(2-Amino-1,3,4-thiadiazol-5-yl)thiomethyl]-7β-[D,2-(3,4-dihydroxyphenyl)-2-[(4-ethyl-2,3-dioxopiperazin-1-yl)carbonylamino]acetamido]-7α-formamidoceph-3-em-4-carboxylate

Sodium 3-[(2-chloroacetamido-1,3,4-thiadiazol-5-yl)thiomethyl]-7β-[D,2-(3,4-dihydroxyphenyl)-2-[(4-ethyl-2,3-dioxopiperazin-1-yl)carbonylamino]acetamido]-7α-formamidoceph-3-em-4-carboxylate (43mg, 0.05mmol) was dissolved in water (3ml) containing sodium N-methyldithiocarbamate (13.5mg, 0.1mmol). After 2.5h, the mixture was washed with ethyl acetate, the aqueous phase separated and concentrated. Chromatography of the residue on "Diaion" HP20SS afforded the title product (18mg, 46%); $\nu_{max}$ (KBr) 3305, 1770, 1705, 1675, and 1605cm$^{-1}$; $\delta_H$ ($D_2O$) (major rotamer) 1.16 (3H, t, J 7.2Hz), 2.98 and 3.39 (2H, ABq, J 16.9Hz), 3.47 (2H, q, J 7.2Hz), 3.6-3.8 (3H, m), 3.9-4.1 (2H, m), 4.23 (1H, d, J 13.7Hz, lower field arm of ABq), 5.24 (1H, s), 5.31 (1H, s), 6.8-7.0 (3H, m) and 8.09 (1H, s). [F.A.B. (+ve ion) (thioglycerol) MH$^+$ 743].

Example 14

Sodium 7β-[D,2-(3,4-Dihydroxyphenyl)-2-[(4-ethyl-2,3-dioxopiperazin-1-yl)carbonylamino]acetamido]-7α-formamido-3-[(2-formamido-1,3,4-thiadiazol-5-yl)thiomethyl]-ceph-3-em-4-carboxylate

Sodium 3-[(2-amino-1,3,4-thiadiazol-5-yl)thiomethyl]-7β-[D,2-(3,4-dihydroxyphenyl)-2-[(4-ethyl-2,3-dioxopiperazin-1-yl)carbonylamino]acetamido]-7α-formamidoceph-3-em-4-carboxylate (15mg, 0.02mmol) was dissolved in water (2ml), carefully acidified to pH 2 with 0.1M $H_2SO_4$ and the mixture lyophilised. A suspension of this acid product in dry dichloromethane (10ml) containing N-methyl-N-(trimethylsilyl)-trifluoroacetamide (56mg, 0.28mmol) was refluxed under argon for 1h, and then stirred at room temperature for 2h. Formic-acetic anhydride (35mg, 0.41mmol) was added and after 1h the title product (7mg, 50%) was isolated as described in Example 10; $\nu_{max}$ (KBr) 3295, 1765, 1705, 1690, 1680, and 1605cm$^{-1}$; $\delta_H$ ($D_2O$) (major rotamer only) 1.16 (3H, t, J 7.3Hz), 3.03 and 3.40 (2H, ABq, J 16.8Hz), 3.45 (2H, m), 3.6-3.7 (2H, m), 3.88 and 4.20 (2H, ABq, J 13.6Hz), 3.9-4.05 (2H, m), 5.25 (1H, s), 5.32 (1H, s), 6.8-7.0 (3H, m), 8.09 (1H, s) and 8.41 (1H, s). [F.A.B. (+ve ion) (thioglycerol) MH$^+$ 772].

Example 15

Sodium 3-[(4-Amino-1,2,4-triazol-3-yl)thiomethyl]-7β-[D-2-(3,4-diacetoxyphenyl)-[2-(4-ethyl-2,3-dioxopiperazin-1-yl)carbonylamino]acetamido]-7α-formamidoceph-3-em-4-carboxylate

(a) Diphenylmethyl 7β-Amino-3-[(4-amino-1,2,4-triazol-3-yl)thiomethyl]-7α-formamidoceph-3-em-4-carboxylate

The title compound was prepared from 7β-amino-7α-formamidocephalosporanic acid and sodium 4-amino-1,2,4-triazol-3-thiolate (J.Ind.Chem.Soc. 1974, 12, 288) as described in Example 1(c); $\nu_{max}$ ($CHCl_3$) 3350, 1775, 1705cm$^{-1}$; $\delta[(CD_3)_2CO]$ (major rotamer only) 2.74 (2H, br s, exchange), 3.66 and 3.71 (2H, ABq, J 16.7Hz), 4.20 and 4.30 (2H, ABq, J13.2Hz), 5.22 (1H, s), 5.71 (2H, s, exchange), 6.93 (1H, s), 7.2-7.7 (10H, m), 8.12 (1H, br s, exchange), 8.25 (1H, d, J 0.9Hz, collapses to s on exchange), and 8.27 (1H, s).

(b) Diphenylmethyl 3-[(4-Amino-1,2,4-triazol-3-yl)thiomethyl]-7β-[D-2-(3,4-diacetoxyphenyl)-[2-(4-ethyl-2,3-dioxopiperazin-1-yl)carbonylamino]acetamido]-7α-formamidoceph-3-em-4-caboxylate

Freshly prepared D-2-(3,4-diacetoxyphenyl)-2-[(4-ethyl-2,3-dioxopiperazin-1-yl)carbonylamino]acetyl chloride [generated by treatment of the corresponding acid (105mg, 0.242mmol) with oxalyl chloride (61.5mg, 0.484mmol) in dry dichloromethane (5ml) containing N,N-dimethylformamide ($^1/_{10}$ drop) for 1h], was taken up in dry dichloromethane (10ml) and added to a stirred solution of diphenylmethyl-7β-amino-3-[-

(4-amino-1,2,4-triazol-3-yl)thiomethyl]-7α-formamidoceph-3-em-4-carboxylate (65mg, 0.121mmol) and pyridine (9.6mg, 0.121mmol) at room temperature. After 1h the mixture was evaporated and the residue taken up in dichloromethane, and then washed successively with dilute hydrochloric acid, saturated brine, dried ($MgSO_4$) and evaporated. The residue was chromatographed on silica gel to give the title compound (60mg, 52%); $\nu_{max}$ ($CHCl_3$) 1770, 1710, 1685cm$^{-1}$; δ[$(CD_3)_2CO$] (major rotamer only) 1.17 (3H, q, J 7.1Hz), 2.26 (6H, s), 3.12 and 3.26 (2H, ABq, J 17Hz), 3.50 (2H, q, J 7.1Hz), 3.60-3.75 and 3.95-4.10 (each 2H, m), 4.21 and 4.48 (2H, ABq, J 13.5Hz), 5.27 (1H, s), 5.71 (1H, d, J 6Hz), 6.89 (1H, s), 7.2-7.7 (13H, m), 8.29 (1H, s), 8.61 (1H, br s), 8.91 (1H, br s), and 10.07 (1H, d, J 6Hz). [F.A.B. (+ve ion) (thioglycerol) $MH^+$ 955].

(c) Sodium 3-[(4-Amino-1,2,4-triazol-3-yl)thiomethyl]-7β-[D-2-(3,4-diacetoxyphenyl)-[2-(4-ethyl-2,3-dioxopiperazin-1-yl)carbonylamino]acetamido]-7α-formamidoceph-3-em-4-carboxylate

A solution of diphenylmethyl 3-[(4-amino-1,2,4-triazol-3-yl)thiomethyl]-7β-[D-2-(3,4-diacetoxyphenyl)-[2-(4-ethyl-2,3-dioxopiperazin-1-yl)carbonylamino]acetamido]-7α-formamidoceph-3-em-4-carboxylate (60mg, 0.063mmol) and anisole (204mg, 1.89mmol) in dry dichloromethane (6ml) at ice bath tenperature was treated with trifluoroacetic acid (216mg, 1.89mmol). The mixture was allowed to warm up to room temperature. After 20min the mixture was evaporated to dryness and the residue triturated with dry ether to give a white solid which was suspended in water and the pH adjusted to 7 with dilute sodium hydrogen carbonate. Chromatography of the resulting solution on Diaion HP20SS and lyophilisation of the product-containing fractions (HPLC monitoring) gave the title compound (11mg, 22%) as a white solid; $\lambda_{max}$ ($H_2O$) 260nm (ε 12,200); $\nu_{max}$ (KBr) 3295, 1765, 1710, 1680, 1605cm$^{-1}$; δ ($D_2O$) (major rotamer only) 1.14 (3H, t, J 7.2Hz), 2.29 (6H, s,), 2.93 and 3.35 (2H, ABq, J 16.9Hz), 3.46 (2H, q, J 7.2Hz), 3.60-3.75 (2H, m), 3.75 and 4.15 (2H, ABq, J 13.4Hz), 3.90-4.05 (2H, m), 5.18 (1H, s), 5.50 (1H, s), 7.2-7.6 (3H, m), and 8.07 (1H, s). [F.A.B. (+ve ion) (thioglycerol) $MH^+$ 811].

## Example 16

Diphenylmethyl 7β-Amino-7α-formamido-3-[(1-formamido-1H-tetrazol-5-yl)thiomethyl]ceph-3-em-4-carboxylate

The title compound was prepared from 7β-amino-7α-formamidocephalosporanic acid and 1-formamido-1H-tetrazol-5-thiol (European Patent Application Publication No. 0 081 971) as described in Example 1(c); $\nu_{max}$ (THF) 3330, 1885, 1730, 1720 and 1695cm$^{-1}$; δ[$(CD_3)_2CO$] (major rotamer only) 2.87 (2H, br s, exchange), 3.63 and 3.74 (2H, ABq, J 17.1Hz), 4.32 and 4.52 (2H, ABq, J 13.3Hz), 5.21 (1H, s), 6.95 (1H, s), 7.3-7.7 (11H, m), 8.12 (1H, br s, exchange), 8.26 (1H, d, J 1.1Hz, collapses to s on exchange) and 8.55 (1H, s). [F.A.B. (+ve ion) (3-nitrobenzyl alcohol) $MNa^+$ 589].

## Example 17

Sodium 7β-[D-2-[(4-Ethyl-2,3-dioxopiperazin-1-yl)carbonylamino]-2-phenylacetamido]-3-[[2-(3,4-dihydroxybenzoylamino)-1,3,4-thiadiazol-5-yl]thiomethyl]-7α-formamidoceph-3-em-4-carboxylate

Sodium 3-[[2-(3,4-diacetoxybenzoylamino)-1,3,4-thiadiazol-5-yl]thiomethyl]-7β-[2-[(4-ethyl-2,3-dioxopiperazin-1-yl)carbonylamino]-2-phenylacetamido]-7α-formamidoceph-3-em-4-carboxylate (93mg; 0.1mmol) in water (5ml) at pH 7.2 was treated with citrus acetyl esterase (1ml) at ambient temperature. After 3h, the pH was adjusted to 7, diluted with acetone and the mixture concentrated to reduced volume. The residue was chromatographed on "Diaion" HP20SS resin to afford, after lyophilisation of the product containing fractions, the title product (60mg, 64%); $\nu_{max}$ 3400, 1775, 1710, 1675, 1600 and 1520cm$^{-1}$; δ ($D_2O$) (major rotamer only) 1.22 (3H, t, J 7.2Hz), 3.09 and 3.43 (2H, ABq, J 16.8Hz), 3.56 (2H, q, J 7.2Hz), 3.75 (2H, m), 4.09 (2H, m), 4.24 and 4.39 (2H, ABq, J 13.2Hz), 5.31 (1H, s), 5.64 (1H, s), 7.05 (1H, m), 7.3-7.6 (7H, m), and 8.18 (1H, s).

Example 18

Sodium 7β-[D-2-[(4-Ethyl-2,3-dioxopiperazin-1-yl)carbonylamino]-2-phenylacetamido]-7α-formamido-3-([2-[(5-hydroxy-4-oxo-1,4-dihydropyridin-2-yl)carbonylamino]-1,3,4-thiadiazol-5-yl]thiomethyl)ceph-3-em-4-carboxylate

(a) Diphenylmethyl 7β-[D-2-[(4-Ethyl-2,3-dioxopiperazin-1-yl)carbonylamino]-2-phenylacetamido]-7α-formamido-3-([2-[(4,5-bis(4-methoxybenzyloxy)pyridin-2-yl]carbonylamino]-1,3,4-thiadiazol-5-yl]thiomethyl)-ceph-3-em-4-carboxylate

A solution of 4,5-bis(4-methoxybenzyloxy)pyridine-2-carboxylic acid (58mg, 0.147mmol) in dichloromethane (2ml) containing N-ethyldiisopropylamine (0.025ml, 0.147mmol) was cooled under argon to -76° C, methanesulphonyl chloride (0.011ml, 0.147mmol) added, and the resulting solution stirred at -10° C for 30 min. This solution was then added with stirring to diphenylmethyl 3-[(2-amino-1,3,4-thiadiazol-5-yl)-thiomethyl]-7β-[D-2-[(4-ethyl-2,3-dioxopiperazin-1-yl)carbonylamino]-2-phenylacetamido]-7α-formamido-ceph-3-em-4-carboxylate (50mg, 0.058mmol) in anhydrous tetrahydrofuran (1ml) containing pyridine (0.012ml, 0.147mmol). After 20h the reaction mixture was diluted with ethyl acetate and washed successively with dilute hydrochloric acid, dilute aqueous sodium hydrogen carbonate solution, brine, dried ($Na_2SO_4$), filtered and evaporated to afford the title product as a white solid (70mg, 95%); $\nu_{max}$ ($CH_2Cl_2$) 3300, 1790, 1715, 1690 and 1620cm$^{-1}$; $\delta_H$ [$(CD_3)_2CO$ (major rotamer)] 1.16 (3H, t, J 7.2Hz), 3.18 and 3.30 (2H, ABq, J 16Hz), 3.49 (2H, q, J 7.2Hz), 3.67 (2H, m), 3.79 (6H, s), 4.05 (2H, m), 4.24 and 4.62 (2H, ABq, J 13.4Hz), 5.31 (2H, AA'), 5.29 and 5.36 (2H, ABq, J 12.3Hz, overlaps 1H, s), 5.75 (1H, d, J 6.9Hz), 6.84 (1H, s), 6.90-7.70 (23H, m), 7.90 (1H, s), 8.29 (1H, s), 8.37 (1H, s), 8.53 (1H, s, exch.), 8.67 (1H, s), 10.03 (1H, d, J 6.6Hz), 11.32 (1H, br s, exch.). [F.A.B. (+ve ion) (thioglycerol) $MH^+$ 1233].

(b) Sodium 7β-[D-2-[(4-Ethyl-2,3-dioxopiperazin-1-yl)carbonylamino]-2-phenylacetamido]-7α-formamido-3-([2-[(5-hydroxy-4-oxo-1,4-dihydropyridin-2-yl)carbonylamino]-1,3,4-thiadiazol-5-yl]thiomethyl)ceph-3-em-4-carboxylate

To a solution of diphenylmethyl 7β-[D-2-[(4-ethyl-2,3-dioxopiperazin-1-yl)carbonylamino]-2-phenylacetamido]-7α-formamido-3-([2-[(4,5-bis(4-methoxybenzyloxy)pyridin-2-yl]carbonylamiono]-1,3,4-thiadiazol-5-yl]thiomethyl)ceph-3-em-4-carboxylate (65mg, 0.053mmol) in dichloromethane (3.5ml) at 0° C was added anisole (0.417ml, 0.38mmol) followed by trifluoroacetic acid (0.296ml, 0.38mmol). After 45 min the reaction mixture was diluted with toluene and evaporated. The residue was diluted with toluene and evaporated; this was repeated. Trituration with ether gave a solid which was suspended in water and the pH adjusted to 7.0 by addition of saturated aqueous sodium hydrogen carbonate solution. The resulting solution was purified by "Diaion HP20SS" resin chromatography eluting with water and water-acetone mixtures. The product-containing fractions were combined, concentrated and lyophilised to afford the title product (35mg, 78%); $\nu_{max}$ (KBr) 3400, 3300, 1770, 1710, 1675 and 1610cm$^{-1}$; $\delta_H$ [($D_2O$) (major rotamer)] 1.14 (3H, t, J 7.2Hz), 2.91 and 3.25 (2H, ABq, J 16.7Hz), 3.45 (2H, m), 3.64 (2H, m), 3.89 and 4.23 (2H, ABq, J 13.6Hz), 3.95 (2H, m), 5.23 (1H, s), 5.47 (1H, s), 6.44 (1H, s), 7.30-7.55 (5H, m), 7.67 (1H, s), 8.08 (1H, s). [F.A.B. (+ve ion) (thioglycerol) $MH^+$ $MNa^+$ 849, 871].

The starting 4,5-bis(4-methoxybenzyloxy)pyridine-2-carboxylic acid was prepared as follows:

a) 2-Hydroxymethyl-5-(4-methoxybenzyloxy)-4-pyrone

Kojic acid (Fluka Chemie AG) (10.0g, 70.4mmol) and 4-methoxybenzyl chloride (10.5ml, 12.1g, 77.4mmol) were dissolved in dry DMF (50ml) and freshly powdered $K_2CO_3$ (19.5g, 140.7mmol) was added. The heterogeneous mixture was stirred for 2 hours then heated at 60° C for a further 3 hours. When cool the mixture was poured into water (300ml) and the aqueous phase extracted with $CH_2Cl_2$ (3 x 80ml). The combined organic phase was washed with water and brine, then dried over $MgSO_4$. Evaporation under

reduced pressure gave a solid residue which was recrystallised from $CHCl_3$ to give the title compound as a cream-coloured crystalline solid (10.9g); $\nu_{max}$ (KBr) 3308, 1653, 1642, 1615 and 1588cm$^{-1}$; $\delta_H$ ($CDCl_3$) 7.51 (1H, s), 7.30 (2H, d, J 8.6Hz), 6.88 (2H, d, J 8.6Hz), 6.51 (1H, s), 4.96 (2H, s), 4.64 (2H, d, J 6.0Hz), 3.87 (1H, t, J 6.0Hz), and 3.80 (3H, s).

b) 5-(4-Methoxybenzyloxy)-4-pyrone-2-carboxylic acid

2-Hydroxymethyl-5-(4-methoxybenzyloxy)-4-pyrone (5.0g, 19.1mmol) was dissolved in acetone (250ml) with gentle warming and the resulting solution cooled in an ice bath. Jones reagent [prepared from concentrated sulphuric acid (4.7ml), water (15ml), and $CrO_3$ (5.37g)] was added dropwise over 5 minutes. The mixture was stirred for 1 hour, the cooling bath removed and stirring continued for a further 1 hour. Methanol (50ml) was then added. The green precipitate was removed by filtration through Kieselguhr and the filtrate evaporated to dryness under reduced pressure. The solid residue was triturated with methanol and the product collected by filtration and washed with acetone and ether.

The title compound was thus obtained as a pale green solid (1.4g); $\nu_{max}$ (KBr) 1740, 1617 and 1595cm$^{-1}$; $\delta_H$ ($CDCl_3/CD_3COCD_3$) 7.82 (1H, s), 7.35 (2H, m), 7.15 (1H, s), 6.90 (1H, s), 5.06 (2H, s), and 3.81 (3H, s).

c) 5-(4-Methoxybenzyloxy)-4-oxo-1,4-dihydropyridine-2-carboxylic acid

5-(4-Methoxybenzyloxy)-4-pyrone-2-carboxylic acid (1.05g, 3.80mmol) was suspended in .880 ammonia solution (30ml) and the mixture heated to reflux for 2 hours. A further 15ml of .880 ammonia solution was then added and heating continued for 1 hour. After cooling the solution was acidified to pH 1 using 5M aqueous HCl. The resulting off-white solid was obtained by filtration and washed with acetone and then ether. After drying in vacuo the title compound was obtained as a beige solid (813mg); $\nu_{max}$ (KBr) 1658 and 1590cm$^{-1}$; $\delta_H$ ($CD_3SOCD_3/D_2O$) 7.91 (1H, s), 7.39 (2H, d, J 8.6Hz), 7.22 (1H, s), 6.96 (2H, d, J 8.6Hz), 5.11 (2H, s), and 3.76 (3H, s).

d) 4-Methoxybenzyl 4,5-Bis(4-methoxybenzyloxy)pyridine-2-carboxylate

5-(4-Methoxybenzyloxy)-4-oxo-1,4-dihydropyridine-2-carboxylic acid (700mg, 2.54mmol) was dissolved in DMF (30ml) and 4-methoxybenzyl chloride (1.72ml, 1.99g, 12.72mmol) was added. Freshly powdered $K_2CO_3$ (3.52g, 25.4mmol) was added and the heterogeneous mixture stirred and heated to 60°C for 18 hours. The mixture was then diluted with EtOAc (150ml) and water (150ml). The organic phase was separated and washed three times with water, once with 0.5M aqueous HCl, once with saturated aqueous sodium hydrogen carbonate then with water and brine and dried over $MgSO_4$. The solvent was removed under reduced pressure and the residue chromatographed on silica gel. Elution with ethyl acetate/hexane mixtures provided the title compound as a white crystalline solid (794mg); $\nu_{max}$ (KBr) 1702 and 1612cm$^{-1}$; $\delta_H$ ($CDCl_3$) 8.24 (1H, s), 7.72 (1H, s), 7.42-7.26 (6H, m), 6.92-6.85 (6H, m), 5.33 (2H, m), 5.17 (2H, s), 5.15 (2H, s), 3.81 (3H, s), 3.80 (3H, s), and 3.79 (3H, s).

e) 4,5-Bis(4-Methoxybenzyloxy)pyridine-2-carboxylic acid

4-Methoxybenzyl 4,5-di(4-methoxybenzyloxy)pyridine-2-carboxylate (300mg, 0.588mmol) was dissolved in THF (15ml) and 2M aqueous potassium hydroxide (6ml) was added. The mixture was stirred and heated to reflux for 90 minutes. The THF was removed by evaporation under reduced pressure and the concentrated aqueous residue diluted with water and acidified to pH 1 with concentrated aqueous HCl.

The resulting white precipitate was separated by filtration and dried over $P_2O_5$ in vacuo to give the title compound (247mg); $\nu_{max}$ 1732 and 1612cm$^{-1}$; $\delta_H$ ($CD_3SOCD_3$) 8.42 (1H, s), 7.92 (1H, s), 7.39 (4H, m), 6.96 (4H, m), 5.37 (2H, s), 5.28 (2H, s), 3.76 (3H, s), and 3.75 (3H, s). [F.A.B. (+ve ion, Thioglycerol) $MH^+$ 396].

Example 19

Minimum Inhibitory Concentration (MIC) values of compounds of the invention against Enterobacter cloacae P99 and Pseudomonas aeruginosa Charlier were determined by serial dilution in agar. The plates were inoculated with $10^4$ colony forming units and incubated overnight at 37° C. The MIC values recorded in Table 1 were the lowest concentration of antibiotic to inhibit growth. Comparative data for 7β-[2-(3,4-diacetoxyphenyl)-2-[4-ethyl-2,3-dioxopiperazin-1-yl)carbonylamino]acetamido]-7α-formamido-3-[1-methyl-1H-tetrazol-5-yl) thiomethyl]ceph-3-em-4-carboxylic acid, sodium salt (compound A), disclosed in European Patent Application No.82303821.1 (Publication Number 0 071 395; Example 57), are also given.

Table 1

MIC data

| Organism | MIC (µg/ml) | | |
|---|---|---|---|
| | Example 1 | Example 2 | Compound A |
| Enterobacter cloacae P99 | 2 | 4 | 8 |
| Pseudomonas aeruginosa Charlier | 2 | 2 | 4 |

## Claims

1. A compound of formula (I) or a salt thereof:

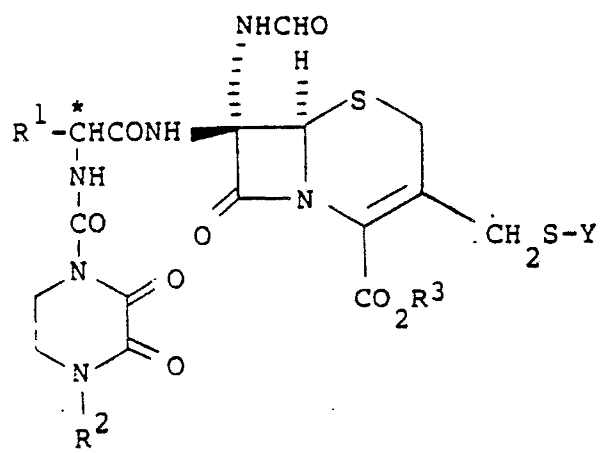

(I)

wherein Y is a group of formula (a), (b) or (c):

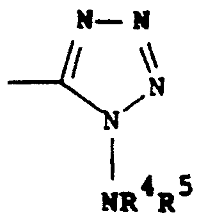

(a)

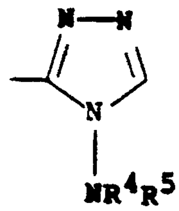

(b)

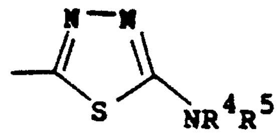

(c)

wherein $R^4$ is hydrogen, optionally substituted $C_{1-8}$ alkyl, formyl, optionally substituted $C_{1-6}$ alkylcarbonyl, optionally substituted $C_{1-6}$ alkoxycarbonyl, arylcarbonyl, aryl $C_{1-6}$ alkylcarbonyl, aryl $C_{1-6}$ alkoxycarbonyl or a group of the formula:

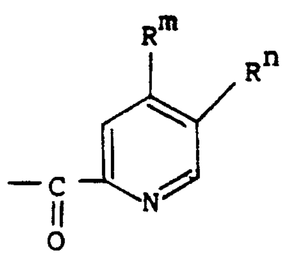

wherein $R^m$ and $R^n$ are hydroxy or protected hydroxy; $R^5$ is hydrogen or $C_{1-8}$ alkyl; or $R^4$ and $R^5$ together form a group $-(CH_2)_n-$ wherein n is an integer having the value 2 to 7, or a group $-(CH_2)_p$ X $(CH_2)_q-$ wherein X is O, S or a group NR wherein R is H or $C_{1-6}$ alkyl and p and q are integers which may be the same or different, each having the value 1 to 5 and wherein p + q has the value 2 to 6; or $R^4$ and $R^5$ together form a $C_{1-6}$ alkylidene or arylalkylidene group containing up to 6 carbon atoms in the alkylidene moiety; $R^1$ is phenyl, substituted phenyl, cyclohexenyl, cyclohexadienyl, or a 5- or 6-membered heterocyclic ring containing up to three hetero-atoms selected from oxygen, sulphur or nitrogen, optionally substituted with hydroxy, amino, halogen, $C_{1-6}$ alkylamino, di($C_{1-6}$ alkyl)amino, or $C_{1-6}$ alkoxy; $R^2$ is $C_{1-8}$ alkyl; and $R^3$ is hydrogen or a readily removable carboxy protecting group.

2. A compound of the formula (IA) or a pharmaceutically acceptable salt or pharmaceutically acceptable in vivo hydrolysable ester thereof:

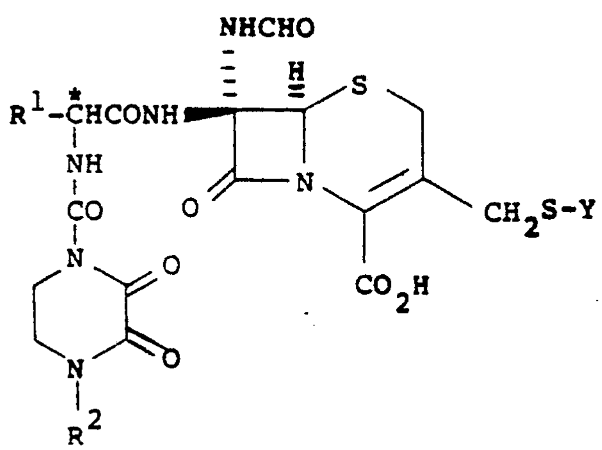

(IA)

wherein $R^1$, $R^2$, and Y are as defined with respect to formula (I) as defined in Claim 1.

3. A compound as claimed in Claim 1 or Claim 2 in which $R^1$ is phenyl or substituted phenyl.

4. A compound as claimed in Claim 3 in which the substituted phenyl group is substituted with up to three groups selected from hydroxy and amino, the hydroxy and amino groups being optionally protected.

5. A compound as claimed in any one of Claims 1 to 4 in which $R^2$ is ethyl.

6. A compound as claimed in any one of Claims 1 to 5 in which $R^4$ is hydrogen, $C_{1-6}$ alkyl, formyl, $C_{1-4}$ alkylcarbonyl optionally substituted by halogen, phenylcarbonyl optionally substituted in the phenyl ring with up to two hydroxy groups or protected hydroxy groups or a group of the formula:

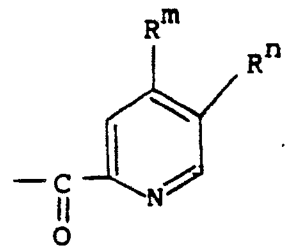

wherein $R^m$ and $R^n$ are hydroxy or protected hydroxy, and $R^5$ is hydrogen; or $R^4$ and $R^5$ together form an alkylidene group containing 2 to 6 carbon atoms in the alkylidene moiety.

7. A compound as claimed in Claim 6 wherein $R^4$ is hydrogen.

8. A compound as claimed in any one of Claims 2-7 selected from the following or a pharmaceutically acceptable salt or pharmaceutically acceptable in vivo hydrolysable ester thereof:

3-[(1-Amino-1H-tetrazol-5-yl)thiomethyl]-7β-[D-2-(3,4-diacetoxyphenyl)-2-[(4-ethyl-2,3-dioxopiperazin-1-yl)-carbonylamino]acetamido]-7α-formamidoceph-3-em-4-carboxylic acid;

3-[(1-Amino-1H-tetrazol-5-yl)thiomethyl]-7β-[D-2-(3,4-dihydroxyphenyl)-2-[(4-ethyl-2,3-dioxopiperazin-1-yl)-carbonylamino]acetamido]-7α-formamidoceph-3-em-4-carboxylic acid;

3-[(2-Chloroacetamido-1,3,4-thiadiazol-5-yl)thiomethyl]-7β-[D-2-[(4-ethyl-2,3-dioxopiperazin-1-yl)-carbonylamino]-2-phenylacetamido]-7α-formamidoceph-3-em-4-carboxylic acid;

3-[(2-Amino-1,3,4-thiadiazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylic acid;

3-[(2-Chloroacetamido-1,3,4-thiadiazol-5-yl)-thiomethyl]-7β-[D,2-(3,4-dihydroxyphenyl)-2-[(4-ethyl-2,3-dioxopiperazin-1-yl)carbonylamino]acetamido]-7α-formamidoceph-3-em-4-carboxylic acid;

3-[(2-Amino-1,3,4-thiadiazol-5-yl)thiomethyl]-7β-[D-2-(3,4-dihydroxyphenyl)-2-[(4-ethyl-2,3-dioxopiperazin-1-yl)carbonylamino]acetamido]-7α-formamidoceph-3-em-4-carboxylic acid;

7β-[D-2-[(4-Ethyl-2,3-dioxopiperazin-1-yl)carbonylamino]-2-phenylacetamido]-7α-formamido-3-[(2-formamido-1,3,4-thiadiazol-5-yl)thiomethyl]ceph-3-em-4-carboxylic acid;

7β-[D-2-(3,4-Dihydroxyphenyl)-2-[(4-ethyl-2,3-dioxopiperazin-1-yl)carbonylamino]acetamido]-7α- formamido-3-[(2-formamido-1,3,4-thiadiazol-5-yl) thiomethyl]ceph-3-em-4-carboxylic acid;

3-[(1-Amino-1H-tetrazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylic acid;

7β-[D-2-[(4-Ethyl-2,3-dioxopiperazin-1-yl)carbonylamino]-2-phenylacetamido]-7α-formamido-3-[(1-isopropylidenamino-1H-tetrazol-5-yl)thiomethyl] ceph-3-em-4-carboxylic acid;

3-[(1-Amino-1H-tetrazol-5-yl)thiomethyl]-7β-[D-2-[(4-ethyl-2,3-dioxopiperazin-1-yl)carbonylamino]-2-(4-hydroxyphenyl)acetamido]-7α-formamidoceph-3-em-4-carboxylic acid;

7β-[D-2-(4-Acetoxyphenyl)-[2-(4-ethyl-2,3-dioxopiperazin-1-yl)carbonylamino]acetamido]-3-[(1-amino-1H-tetrazol-5-yl)thiomethyl]-7α-formamidoceph-3-em-4-carboxylic acid;

7β-[D-2-(3,4-Dihydroxyphenyl)-2-[(4-ethyl-2,3-dioxopiperazin-1-yl)carbonylamino]acetamido]-7α-formamido-3-[(1-methylamino-1H-tetrazol-5-yl) thiomethyl]ceph-3-em-4-carboxylic acid;

3-[[2-(3,4-Diacetoxybenzoylamino)-1,3,4-thiadiazol-5-yl]thiomethyl]-7β-[2-[(4-ethyl-2,3-dioxopiperazin-1-yl)-carbonylamino]-2-phenylacetamido]-7α-formamidoceph-3-em-4-carboxylic acid;

3-[(4-Amino-1,2,4-triazol-3-yl)thiomethyl]-7β-[D-2-(3,4-diacetoxyphenyl)-[2-(4-ethyl-2,3-dioxopiperaz in-1-yl)-carbonylamino]acetamido]-7α-formamidoceph-3-em-4-carboxylic acid;

7β-[D-2-[(4-Ethyl-2,3-dioxopiperazin-1-yl)carbonylamino]-2-phenylacetamido]-3-[[2-(3,4-dihydroxybenzoylamino)-1,3,4-thiadiazol-5-yl]thiomethyl]-7α-formamidoceph-3-em-4-carboxylic acid; and

7β-[D-2-[(4-Ethyl-2,3-dioxopiperazin-1-yl)carbonylamino]-2-phenylacetamido]-7α-formamido-3-([2-[(5-hydroxy-4-oxo-1,4-dihydropyridin-2-yl)carbonylamino]-1,3,4-thiadiazol-5-yl]thiomethyl)ceph-3-em-4-carboxylic acid.

9. A pharmaceutical composition comprising an antibacterially effective amount of a pharmaceutically acceptable compound as claimed in any one of Claims 2 to 8 together with a pharmaceutically acceptable carrier or excipient.

10. A pharmaceutical composition as claimed in Claim 9 which further comprises a β-lactamase inhibitor.

11. A process for the preparation of a compound as claimed in any one of Claims 1 to 8, which process comprises reacting a compound of formula (IX) or a salt thereof:

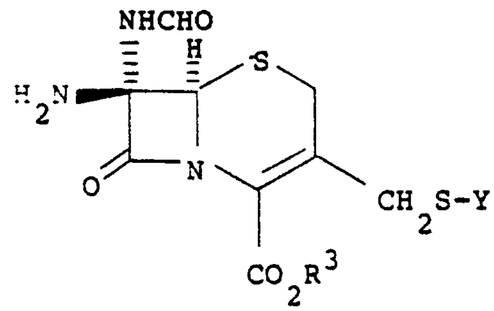

(IX)

wherein $R^3$ and Y are as defined with respect to formula (I) in Claim 1; the 7β-amino group is optionally substituted with a group which permits acylation to take place; and any reactive groups may be protected; with an N-acylating derivative of an acid of formula (X):

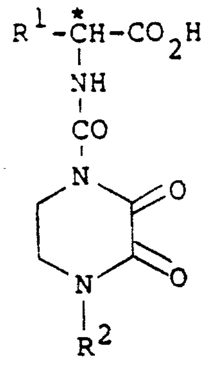

(X)

wherein $R^1$ and $R^2$ are as defined with respect to formula (I) in claim 1, and wherein any reactive groups may be protected; and thereafter, if necessary, carrying out one or more of the following steps:

i) converting a group $R^3$ into another group $R^3$;

ii) removing any protecting groups;

iii) converting the product into a salt.

12. A compound of the formula (IX):

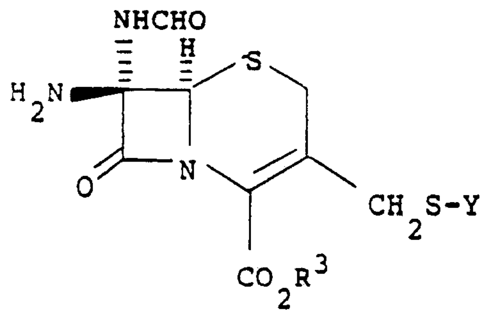

(IX)

wherein $R^3$ and Y are as defined with respect to formula (I) in Claim 1.

13. A compound as claimed in Claim 12 selected from the following:

Sodium 7β-Amino-3-[(1-amino-1H-tetrazol-5-yl)thiomethyl]-7α-formamidoceph-3-em-4-carboxylate;

7β-Amino-3-[(1-amino-1H-tetrazol-5-yl)thiomethyl]-7α-formamidoceph-3-em-4-carboxylic acid;

Diphenylmethyl 7β-Amino-3-[(1-amino-1H-tetrazol-5-yl) thiomethyl]-7α-formamidoceph-3-em-4-carboxylate;

Diphenylmethyl 7β-Amino-7α-formamido-3-[(1-iso-propylideneamino-1H-tetrazol-5-yl)thiomethyl]ceph-3-em-4-carboxylate;

Sodium 7β-Amino-7α-formamido-3-[(1-methylamino-1H-tetrazol-5-yl)thiomethyl]ceph-3-em-4-carboxylate;

7β-Ammonio-7α-formamido-3-[(1-methylamino-1H-tetrazol-5-yl)thiomethyl]ceph-3-em-4-carboxylate;

Diphenylmethyl 7β-Amino-3-[(2-amino-1,3,4-thiadiazol-5-yl)thiomethyl]-7α-formamidoceph-3-em-4-carboxylate;

Diphenylmethyl 7β-Amino-3-[(2-chloroacetamido-1,3,4-thiadiazol-5-yl)thiomethyl]-7α-formamidoceph-3-em-4-carboxylate;

Sodium 7β-Amino-3-[(2-amino-1,3,4-thiadiazol-5-yl)thiomethyl]-7α-formamidoceph-3-em-4- carboxylate;

7β-Amino-3-[(2-amino-1,3,4-thiadiazol-5-yl) thiomethyl]-7α-formamidoceph-3-em-4-carboxylic acid;

Diphenylmethyl 7β-Amino-3-[(4-amino-1,2,4-triazol-3-yl)thiomethyl]-7α-formamidoceph-3-em-4-carboxylate; and

Diphenylmethyl 7β-Amino-7α-formamido-3-[(1-formamido-1H-tetrazol-5-yl)thiomethyl]ceph-3-em-4-carboxylate.

14. The use of a compound as defined in claim 2 for the manufacture of a medicament for the treatment of bacterial infections in animals including humans.